(19) Europäisches Patentamt
European Patent Office
Office européen des brevets

(11) **EP 4 744 579 A1**

(12) **EUROPEAN PATENT APPLICATION**
published in accordance with Art. 153(4) EPC

(43) Date of publication:
**20.05.2026 Bulletin 2026/21**

(21) Application number: 24839830.7

(22) Date of filing: **12.07.2024**

(51) International Patent Classification (IPC):
**A61B 5/00** (2006.01)   **A61K 8/00** (2006.01)
**A61Q 17/04** (2006.01)   **G01N 21/33** (2006.01)
**G01N 21/47** (2006.01)

(52) Cooperative Patent Classification (CPC):
**A61B 5/00; A61K 8/00; A61Q 17/04; G01N 21/33;
G01N 21/47**

(86) International application number:
**PCT/JP2024/025389**

(87) International publication number:
**WO 2025/013942 (16.01.2025 Gazette 2025/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.07.2023 JP 2023115001**

(71) Applicant: **Kao Corporation
Tokyo 103-8210 (JP)**

(72) Inventors:
• **HARYUU, Yasushi
Tokyo 103-8210 (JP)**
• **OKADA, Tomonari
Tokyo 103-8210 (JP)**
• **TAKEDA, Katsuya
Tokyo 103-8210 (JP)**

(74) Representative: **Hoffmann Eitle
Patent- und Rechtsanwälte PartmbB
Arabellastraße 30
81925 München (DE)**

(54) **EVALUATION METHOD, EVALUATION DEVICE, APPLICATION PROGRAM, AND EVALUATION SYSTEM**

(57)     [Object] To evaluate suitability of a topical skin preparation with a sunscreen effect for a skin of a user.

[Solving Means] An evaluation method includes: obtaining reflection light of UV light radiated to a skin of a user to which a topical skin preparation with a sunscreen effect is applied with UV light; estimating a UV protection value of the topical skin preparation with a sunscreen effect for the skin of the user on the basis of the reflection light; and evaluating suitability of the topical skin preparation with a sunscreen effect for the skin of the user by using the UV protection value.

| Formulation | | A | D | E | F |
|---|---|---|---|---|---|
| Form | | Gel | Gel | Gel | Emulsion |
| Formulation type | | O/W | O/W | O/W | W/O |
| Indicated SPF | | 30 | 50 | 50+ | 50+ |
| Application amount(mg/cm$^2$) | | 1 | 1 | 1 | 1 |
| Application part | | Face | Face | Face | Face |
| Estimated SPF value | Subject 1 | 10.5 | 12.1 | 20.0 | 15.4 |
| | Subject 2 | 24.4 | - | 33.9 | 20.2 |
| | Subject 3 | 21.1 | - | - | 52.5 |
| | Subject 4 | 22.7 | 11.4 | - | - |
| | Subject 5 | - | 11.1 | 34.9 | - |
| | Subject 6 | - | 10.1 | 13.9 | 34.3 |
| | Subject 7 | 20.5 | 16.7 | 22.1 | 28.5 |

FIG.6

EP 4 744 579 A1

**Description**

Technical Field

The present invention relates to an evaluation method of estimating a UV protection value when applying a predetermined topical skin preparation with a sunscreen effect to a skin of a user and evaluating suitability of the topical skin preparation with the sunscreen effect for the skin of the user by using an estimation result.

Background Art

There is a technology of evaluating performance of a ultraviolet protective agent with a function of absorbing and/or scattering UV light put on a living body surface (Patent Literature 1).

Citation List

Patent Literature

Patent Literature 1: Japanese Patent Application Laid-open No. 2017-146211

Disclosure of Invention

Technical Problem

By the way, many users report that they sometimes cannot feel UV protection effects (e.g., SPF values) advertised or indicated on the packages or product websites of topical skin preparations with sunscreen effects. Many users are seeking topical skin preparations with sunscreen effects suitable for their own skin. This is the current situation.
The present invention has been made in view of such a problem. The present invention relates to an evaluation method of evaluating suitability of a topical skin preparation with a sunscreen effect for a skin of a user.

Solution to Problem

The present invention relates to an evaluation method including: irradiating a skin of a user to which a topical skin preparation with a sunscreen effect is applied with UV light and obtaining reflection light of the irradiated UV light; and evaluating suitability of the topical skin preparation for the skin of the user on the basis of the reflection light.
Moreover, the present invention relates to an evaluation apparatus including: an obtaining means for irradiating a skin of a user to which a topical skin preparation with a sunscreen effect is applied with UV light and obtaining reflection light of the irradiated UV light; and an evaluation means for evaluating suitability of the topical skin preparation with the sunscreen effect for the skin of the user by using the reflection light.
Moreover, the present invention relates to an application program that causes an evaluation apparatus irradiates a skin of the user to which a topical skin preparation with a sunscreen effect is applied with UV light and evaluates suitability of the topical skin preparation with the sunscreen effect for the skin of the user by using reflection light of the irradiated UV light to operate, the program including:

an obtaining process of irradiating a skin of a user to which a topical skin preparation with a sunscreen effect is applied with UV light and obtaining reflection light of the irradiated UV light; and
a process of evaluating suitability of the topical skin preparation for the skin of the user by using the reflection light.

Moreover, the present invention relates to an evaluation system including: a UV light irradiate means for irradiating a skin of a user to which a topical skin preparation with a sunscreen effect is applied with UV light; a detection means for detecting reflection light of the irradiated UV light; an obtaining means for obtaining the detected reflection light of the UV light; and an evaluation means for evaluating suitability of the topical skin preparation with the sunscreen effect for the skin of the user by using the reflection light.

Advantageous Effects of Invention

In accordance with the method provided by the present invention, it is possible to evaluate suitability of a topical skin preparation with a sunscreen effect for a skin of a user and it is possible to propose the topical skin preparation with the sunscreen effect suitable for the user.

Brief Description of Drawings

[Fig. 1] An image view when capturing a UV skin image.
[Fig. 2] A block diagram of this evaluation apparatus.
[Fig. 3] A diagram describing a method of estimating a UV protection value of a topical skin preparation with a sunscreen effect applied to a skin of a user.
[Fig. 4] A diagram describing the method of estimating the UV protection value of the topical skin preparation with the sunscreen effect applied to the skin of the user.
[Fig. 5] A functional block diagram (outline) related to estimation of the UV protection value.
[Fig. 6] An estimation result according to Embodiment 1.
[Fig. 7] (a) is a UV skin image of Subject 1 and (b) is a UV skin image of Subject 7.
[Fig. 8] An estimation result of Embodiment 2.
[Fig. 9] An estimation result of Embodiment 3.
[Fig. 10] An estimation result of Embodiment 4.
[Fig. 11] A UV skin image of Subject 21.
[Fig. 12] An estimation result including uniformity of a coating film formed.
[Fig. 13] A block diagram of this evaluation system.
[Fig. 14] A view showing a result of evaluating suitability of a single topical skin preparation for the user in another embodiment.
[Fig. 15] A view showing an example of displaying recommended products to the user on the basis of the result of evaluating the suitability in Fig. 14.
[Fig. 16] A view showing a result of classifying users into a plurality of skin types on the basis of measurement results of suitability of certain topical skin preparations and predicting unknown effects of other topical skin preparations in another embodiment.
[Fig. 17] A view showing an example of displaying recommended products to the user for each skin type classified in Fig. 16.
[Fig. 18] A view showing results of predicting unknown effects of other topical skin preparations on the basis of measurement results of suitability levels of certain topical skin preparations as maps in another embodiment.
[Fig. 19] A view showing suitability evaluations based on a relationship between estimated UV protection values and indicated UV protection values between different body sites in another embodiment.
[Fig. 20] A view showing suitability evaluations based on a relationship between estimated UV protection values and indicated UV protection values between different applicators in another embodiment.

Mode(s) for Carrying Out the Invention

Hereinafter, an example of a favorable embodiment of the present invention will be described with reference to the drawings. It should be noted that the drawings of the present embodiment are all for describing technological concepts, configurations, and operations of the present invention, and not limit its configurations to specific ones. Moreover, in all drawings, similar components are denoted by similar reference signs and duplicate descriptions are omitted as appropriate.

The outline of an estimation method in the present embodiment (hereinafter, it may be referred to as this method) will be described.

The evaluation method according to the present embodiment is an evaluation method including: irradiating a skin of a user to which a topical skin preparation with a sunscreen effect is applied with UV light and obtaining reflection light of the irradiated UV light; and evaluating suitability of the topical skin preparation with the sunscreen effect for the skin of the user on the basis of the reflection light. Moreover, the evaluation method according to the present embodiment is an evaluation method including: estimating a UV protection value of the topical skin preparation with the sunscreen effect for the skin of the user on the basis of the reflection light; and evaluating suitability of the topical skin preparation with the sunscreen effect for the skin of the user by using the UV protection value.

The "topical skin preparation with the sunscreen effect" is a composition containing UV protection components. The UV protection components to be contained only need to contain at least either UV absorbing agents or UV scattering agents. Moreover, any type of indication indicating a UV protection effect to be described later can be employed and the numeric value of the indication can be any value. Moreover, examples of the form of the preparation include liquid, gel, semi-solid, solid, foam, mist, and sheet forms. The type of preparation can be any type such as a W/O cosmetic (water-in-oil cosmetic), an O/W cosmetic (oilin-water cosmetic), an emulsion, a gel, a cream, lotion, a sheet, a makeup cosmetic, or a powder type. Alternatively, the preparation may have functions other than the UV protection effect, such as waterproof capability, suitability for use as a makeup base, moisturizing function, skin tone correction, skin condition improvement, or pollen prevention.

The "irradiating a skin of a user with UV light and obtaining reflection light of the irradiated UV light" means irradiating the skin of the user with UV light through a lighting unit and obtaining reflection light reflected on a bare skin of the user or a skin to which the topical skin preparation with the sunscreen effect is applied. For example, it is obtained through a UV camera. In this case, it is sufficient that the intensity of the obtained reflection light can be calculated. Therefore, it means obtaining a skin image (UV skin image) including a region to which the topical skin preparation with the sunscreen effect is applied. In a case of using the lighting unit that radiates light in a plurality of wavelength bands, an optical filter that transmits light in a specific wavelength band may be used for radiating UV light. Moreover, a polarization plate may be provided to allow light polarized in a specific direction to pass therethrough.

The "evaluating suitability of the topical skin preparation with the sunscreen effect for the skin of the user on the basis of the reflection light means using the intensity of the reflection light calculated on the basis of the obtained reflection light and the UV skin image of the reflection light, thereby evaluating the UV protection effect, uniformity, and the like of the coating film formed on the skin by the topical skin preparation with the sunscreen effect applied to the skin of the user. The "evaluation" means performing comparison and evaluation with a predetermined reference, an indication, or the like, thereby providing a similar evaluation result even in a case where an evaluator differs. For example, it means comparing a captured UV skin image with a reference UV skin image or comparing a value with calculated on the basis of the reflection light with a reference value, for example, thereby evaluating suitability of the topical skin preparation with the sunscreen effect for the skin of the user.

The "UV protection value of the topical skin preparation with the sunscreen effect" means indication indicating the amount of ultraviolet radiation prevented from reaching the subject's skin by applying the topical skin preparation with the sunscreen effect. The UV protection value can be a sun protection factor (SPF), a UVA Protection factor of product (UVAPF), UV transmittance, UV transmittance estimated from the UVB region, critical wavelength, absorbance, or any other value. Alternatively, the UV protection value may be a numeric value for the indication, a difference from a predetermined reference value, a percentage, or a ratio. Moreover, the UV protection value may be determined on the basis of which indication to use and which type of evaluation should be performed in accordance with the type of UV protection component contained in a predetermined composition and a wavelength band of ultraviolet light to be radiated.

The "estimating a UV protection value" means estimating an indication of the degree to which ultraviolet rays can be protected against on the basis of the intensity of the reflection light calculated on the basis of the obtained reflection light. For example, it means estimating it on the basis of a predetermined expression to be described later.

The "evaluating suitability of the topical skin preparation with the sunscreen effect for the skin of the user by using the UV protection value" means evaluating whether the topical skin preparation with the sunscreen effect is suitable for the skin of the user or whether the function of the topical skin preparation with the sunscreen effect can be provided by using comparison with a UV protection value estimated in a case where the same topical skin preparation with a sunscreen effect is applied to another user, the uniformity of the coating film formed on the skin by the topical skin preparation with the sunscreen effect calculated on the basis of a coefficient of variation of the estimated UV protection value, or the like.

Here, suitability of the topical skin preparation with the sunscreen effect for the skin of the user will be described.

The suitability of the topical skin preparation with the sunscreen effect for the skin of the user means whether or not the topical skin preparation with the sunscreen effect is compatible with the skin of the user. The suitability is suitability of the UV protection effect of the topical skin preparation with the sunscreen effect, suitability of an application state formed on the skin, suitability with respect to skin conditions (e.g., sensitive skin) of the user, suitability with respect to a use experience that the user seeks, or the like. The suitability may be any one item or may be a plurality of items. Specifically, as to the suitability of the UV protection effect, the suitability is whether the UV protection value estimated when applying the topical skin preparation with the sunscreen effect to the skin of the user meets a UV protection value expected by the user, whether a UV protection value equivalent to the published UV protection value (e.g., SPF value) of the topical skin preparation with the sunscreen effect is estimated as an estimated UV protection value, or the like. Moreover, as to the suitability of the application state of the topical skin preparation with the sunscreen effect, the suitability is the uniformity of the coating film formed on the skin of the user by the topical skin preparation with the sunscreen effect, the application easiness of the topical skin preparation with the sunscreen effect to the skin, or the like. Moreover, the suitability is the presence/absence of irritations on the skin of the user after the topical skin preparation with the sunscreen effect is applied, for example. Moreover, the suitability with respect to the user's use experience is preference of the use experience based on the user's input result of a questionnaire after the topical skin preparation with the sunscreen effect is applied, for example. By evaluating such suitability, it is possible to propose a topical skin preparation with a sunscreen effect optimal for the skin of the user.

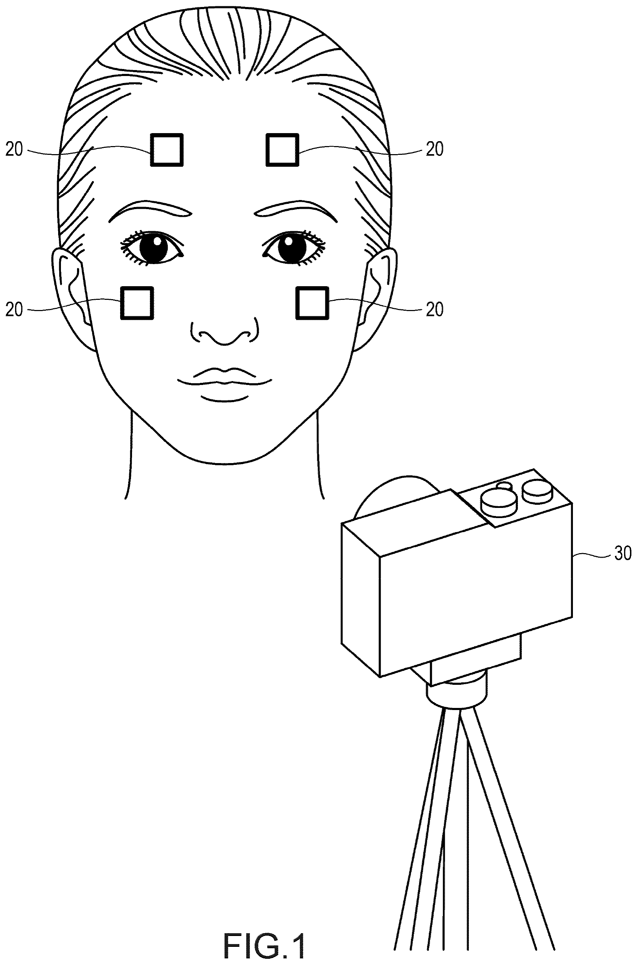

In the present embodiment, as shown in Fig. 1, a topical skin preparation 20 with a sunscreen effect is applied to a skin of a user (here, a user's face). In the present embodiment, it is applied to a total of four points, two for the forehead and one for each of the left and right cheeks. It should be noted that the positions to which the topical skin preparation 20 with the sunscreen effect is applied and the number of positions shown in Fig. 1 are exemplary. Other positions and number are possible. In the present embodiment, UV protection values of four types of compositions are estimated at a time and compared and evaluated by applying different compositions to the four application positions shown in Fig. 1, though not limited thereto. For example, since there are often differences in skin texture, wrinkles, and the like between cheeks and forehead, an application state of the topical skin preparation 20 with the sunscreen effect may differ depending on skin conditions. Therefore, the topical skin preparation 20 with the sunscreen effect may be applied to sites similar in the skin conditions and perform estimation and evaluation. With this configuration, it is possible to perform the comparison in similar skin conditions. Therefore, the composition comparison can be performed clearly. Moreover, for example, the skin conditions may differ between the left cheek and the right cheek. Therefore, for example, multiple kinds (e.g., two kinds) of compositions may be applied into the left cheek. Moreover, to avoid skin condition differences, a single kind of composition may be applied to the same site and its UV protection value may be estimated, the preparation may be removed by face washing, and then a single different kind of preparation may be applied to the same site and its UV protection value may be estimated. Any way of application and evaluation is possible. However, applying topical skin preparations 20 with multiple kinds of sunscreen effects at a time and evaluating them reduces the total evaluation time. Moreover, it is favorable to apply topical skin preparations 20 with multiple kinds of sunscreen effects to different regions at a time and evaluate them because the evaluation can be performed in the same skin conditions (e.g., the possibility that face washing performed every time the composition is changed may change the skin conditions can be avoided).

As described above, the positions to which the topical skin preparation 20 with the sunscreen effect is applied and the type (number) or the like of the topical skin preparation 20 with the sunscreen effect to be applied at a time are not limited. Optimal application position and number of applications only needs to be determined depending on the skin conditions of the user, the type (number) of the topical skin preparation 20 with the sunscreen effect, a time required for evaluation, and the like.

In the present embodiment, as a method of obtaining reflection light of UV light with which the skin of the user (face) to which the topical skin preparation 20 with the sunscreen effect is applied is irradiated, a captured image obtained by imaging the skin of the user (face) to which the topical skin preparation 20 with the sunscreen effect is applied is obtained by a UV camera 30. Upon imaging, UV light is radiated toward the skin of the user from a lighting unit 50 (not shown) capable of radiating light in a UV wavelength band. With this configuration, it is possible to image the skin of the user under UV irradiation.

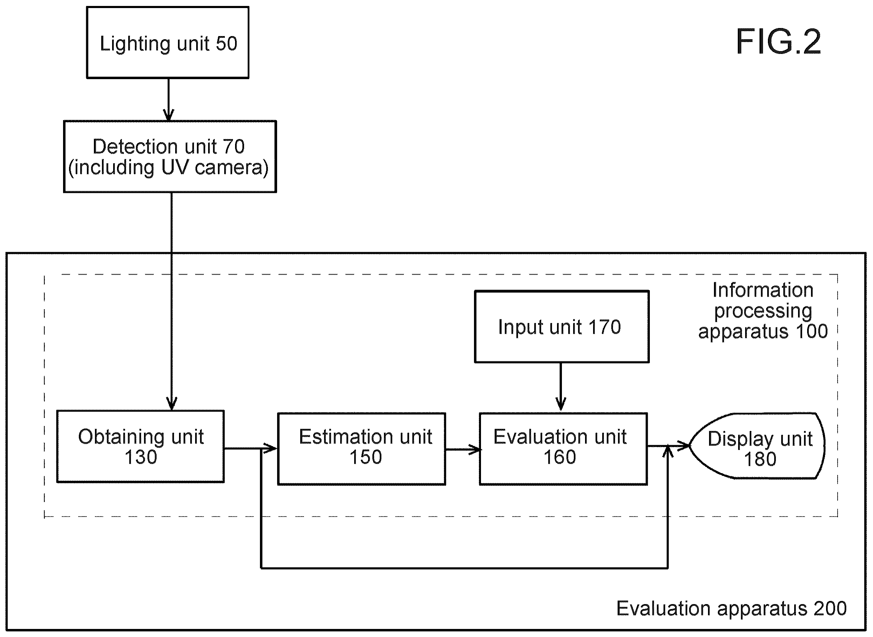

An estimation method for the UV protection value of a coating film of the topical skin preparation with the sunscreen effect applied to the skin of the user will be described with reference to Figs. 2 to 5.

<Evaluation Apparatus 200>

Fig. 2 is a conceptual diagram of an evaluation apparatus 200. This evaluation method enables evaluation of the suitability of the topical skin preparation 20 with the sunscreen effect for the skin of the user by using the evaluation apparatus 200 shown in Fig. 2.

The evaluation apparatus 200 in the present embodiment is constituted by an obtaining unit 130, an estimation unit 150, and an evaluation unit 160. Moreover, the evaluation apparatus 200 includes an information processing apparatus 100 capable of executing various types of processing. The information processing apparatus 100 includes the obtaining unit 130, the estimation unit 150, and the evaluation unit 160. The information processing apparatus 100 is a general-purpose personal computer. The information processing apparatus 100 includes an input unit 170 such as a keyboard, a touch panel, and a pointing device, an arithmetic processing unit (e.g., a CPU or the like), a storage unit, and the like. Moreover, the information processing apparatus 100 favorably includes the input unit 170 and a display unit 180 (including a display device). However, the display unit 180 may be provided to an information processing terminal provided outside the information processing apparatus 100, such as a smartphone, and connected via a network. Moreover, although it is unnecessary to provide them in the evaluation apparatus 200, the lighting unit 50 and a detection unit 70 are necessary because a UV skin image obtained by imaging under UV irradiation is used in this evaluation method as described above. It is assumed that the detection unit 70 includes the above-mentioned UV camera 30.

The obtaining unit 130 is a means (obtaining means) for irradiating the skin of the user to which the topical skin preparation with the sunscreen effect is applied with UV light and obtaining reflection light of the irradiated UV light. The obtaining unit 130 obtains it from the detection unit 70 via a network, for example.

The estimation unit 150 is a means (estimation means) for estimating a UV protection value of the topical skin preparation 20 with the sunscreen effect for the skin of the user on the basis of the UV skin image. An estimation method by the estimation means will be described later.

The evaluation unit 160 is a means (evaluation means) for evaluating the suitability of the topical skin preparation 20 with the sunscreen effect for the skin of the user by displaying data obtained by the obtaining unit 130 on the display unit 180 or using the UV protection value estimated by the estimation unit 150. The evaluation method by the evaluation means will be described later. An evaluation result by the evaluation unit 160 is favorably adjusted to have a display format so that an evaluator can easily understand it through the display unit 180. It should be noted that the "evaluator" set forth herein includes both a store clerk and a user.

The input unit 170 is a means (input means) for the user to input an answer to a predetermined questionnaire.

The display unit 180 is capable of, for example, rearranging the estimation result by the estimation unit 150, the evaluation result by the evaluation unit 160, or the like as appropriate or displaying them as a graph so that the user or the evaluator can easily understand the estimation result and evaluation result.

The storage unit of the information processing apparatus 100 has stored an application program for executing the estimation method and the evaluation method to be described later in the information processing apparatus 100, i.e., realizing the obtaining unit 130, the estimation unit 150, and the evaluation unit 160. The application program causes an arithmetic processing unit to estimate a UV protection value by using data obtained by the obtaining unit 130. The application program causes an arithmetic processing unit to execute evaluation and display an evaluation result.

It is assumed that the lighting unit 50 emits light in a wavelength band including UV light with wavelengths of 280 nm or more, 400 nm or less. It should be noted that if predetermined UV light intensity can be obtained, for example, a lighting unit that emits light in a broad wavelength band (including a UV band) and a lighting unit that emits light in a narrow wavelength band (in the UV band) can be both used.

It should be noted that since the lighting unit 50 and the detection unit 70 are devices required to irradiate the skin of the user to which the topical skin preparation 20 with the sunscreen effect is applied with UV light in the evaluation apparatus 200 and obtain reflection light of the irradiated UV light, the lighting unit 50 and the detection unit 70 are shown in Fig. 2.

<Estimation Method for UV Protection Value>

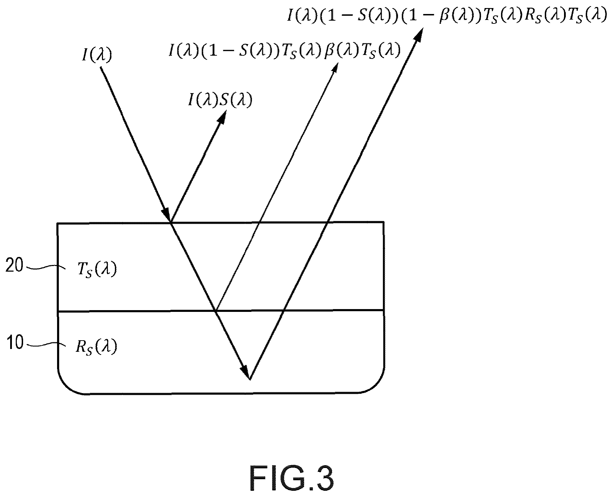

In the present embodiment, as shown in Fig. 3, a topical skin preparation 20 with a sunscreen effect is applied to a surface of a skin of the user 10, the skin of the user 10 is irradiated with UV light (incident light), and its reflection light is detected. Intensity of the incident light is denoted by $I(\lambda)$. Here, intensity $I_R(\lambda)$ of the reflection light is represented by Expression (1) below using the topical skin preparation 20 with the sunscreen effect shown in Fig. 3 and a surface model of the skin of the user 10.

[Formula 1]

$$I_R(\lambda) = I(\lambda)S(\lambda) + I(\lambda)(1-S(\lambda))T_s^2(\lambda)\beta(\lambda) + \\ I(\lambda)(1-S(\lambda))(1-\beta(\lambda))T_s^2(\lambda)R_s(\lambda)$$

$$\ldots \text{Expression (1)}$$

Where $S(\lambda)$ is a percentage of incident light with a wavelength $\lambda$ reflected/scattered on the surface of the topical skin preparation 20 with the sunscreen effect. $(1-S(\lambda))$ is a percentage of the incident light with the wavelength $\lambda$ not reflected/scattered on the surface of the topical skin preparation 20 with the sunscreen effect. $T_s^2(\lambda)$ is a percentage of the incident light with the wavelength $\lambda$ not absorbed by the topical skin preparation 20 with the sunscreen effect, but reaching the interface to the skin of the user 10. $\beta(\lambda)$ is a percentage of the incident light with the wavelength $\lambda$ reflected on the interface between the skin of the user 10 and the topical skin preparation 20 with the sunscreen effect. $R_s(\lambda)$ is a percentage of the incident light with the wavelength $\lambda$ reflected on the skin of the user 10.

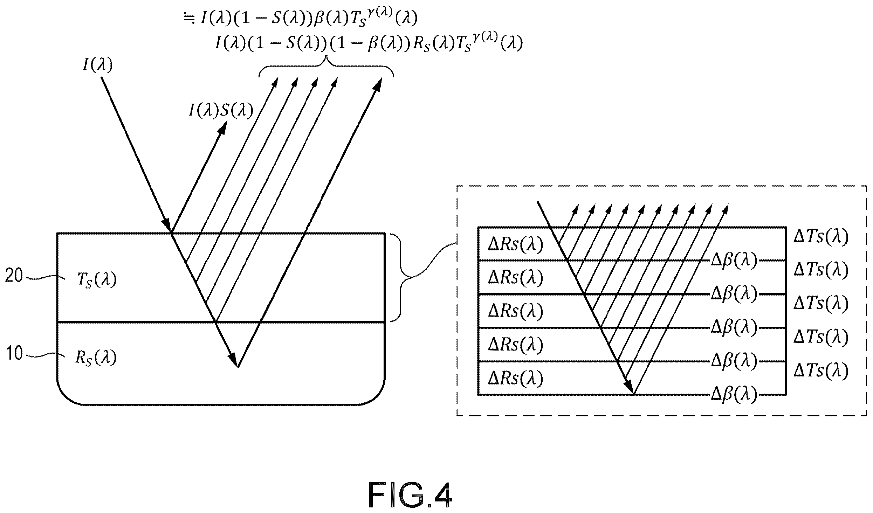

Expression (1) above holds only when the topical skin preparation 20 with the sunscreen effect forms a homogeneous layer without scattering, separated from the skin of the user 10 as shown in Fig. 3. However, in reality, it is considered that a layered structure as shown in Fig. 4 has been formed due to penetration of the topical skin preparation 20 with the sunscreen effect into the skin of the user 10 and scattering properties of the topical skin preparation 20 with the sunscreen effect itself, such as incorporation of UV scattering agents. At that time, it is considered that the optical path length in the topical skin preparation 20 with the sunscreen effect has varied. The intensity $I_R(\lambda)$ of the reflection light can be approximated by Expression (2) below.

[Formula 2]

$$I_R(\lambda) \fallingdotseq I(\lambda)S(\lambda) + I(\lambda)(1-S(\lambda))T_s^{\gamma(\lambda)}(\lambda)\beta(\lambda) + \\ I(\lambda)(1-S(\lambda))(1-\beta(\lambda))T_s^{\gamma(\lambda)}(\lambda)R_s(\lambda)$$

$$\ldots \text{Expression (2)}$$

Where $\gamma(\lambda)$ is a correction parameter for the transmittance at wavelength $\lambda$, accounting for the variation in the optical path length.

The "transmittance T($\lambda$) of the UV light to the topical skin preparation 20 with the sunscreen effect," which is a percentage of the incident light with the wavelength $\lambda$ not absorbed/scattered, for example, by the topical skin preparation 20 with the sunscreen effect but reaches the skin of the user 10 is estimated. The transmittance T($\lambda$) of the UV light to the topical skin preparation 20 with the sunscreen effect is represented by T($\lambda$) = (1-S($\lambda$)) (1-$\beta$($\lambda$))T$_s$($\lambda$).

Here, a reflectance R($\lambda$) that is a ratio of intensity I(A) of the incident light with the wavelength $\lambda$ to the intensity I$_R$($\lambda$) of the reflection light is represented by R($\lambda$) = I$_n$($\lambda$)/I($\lambda$). Expression (3) below is obtained by substituting the right side of Expression (2) for I$_R$($\lambda$) of this expression.

[Formula 3]

$$R(\lambda) = I_R(\lambda)/I(\lambda) = S(\lambda) + (1-S(\lambda))\, T_s^{\gamma(\lambda)}(\lambda)\, \beta(\lambda) + (1-S(\lambda))(1-\beta(\lambda))\, T_s^{\gamma(\lambda)}(\lambda) R_s(\lambda)$$

$$\dots \text{Expression (3)}$$

By rewriting Expression (3), Expression (4) below is obtained.

[Formula 4]

$$\{(1-S(\lambda))(1-\beta(\lambda))\, T_s(\lambda)\}^{\gamma(\lambda)}$$

$$= \frac{R(\lambda) - S(\lambda)}{\beta(\lambda) + (1-\beta(\lambda)) R_s(\lambda)} = (1-S(\lambda))^{\gamma(\lambda)-1}(1-\beta(\lambda))^{\gamma(\lambda)}$$

$$\dots \text{Expression (4)}$$

By multiplying both sides of Expression (4) by 1/y($\lambda$) yields, the left side matches the right side of the above-mentioned calculation expression for T($\lambda$). That is, Expression (5) below is obtained.

[Formula 5]

$$T_S(\lambda) = \left\{ \frac{R(\lambda) - S(\lambda)}{\beta(\lambda) + (1-\beta(\lambda)) R_S(\lambda)} \right\}^{\frac{1}{\gamma(\lambda)}} (1 - S(\lambda))^{\frac{\gamma(\lambda)-1}{\gamma(\lambda)}} (1 - \beta(\lambda))^{\gamma(\lambda)}$$

$$\dots \text{Expression (5)}$$

Here, for the sake of description, a process of radiating UV light (incident light) from the lighting unit 50 is defined as Process 1, a process of detecting reflection light of the UV light radiated in Process 1 is defined as Process 2, and a process of estimating transmittance of the topical skin preparation 20 with the sunscreen effect to UV light on the basis of a detection result of Process 2 and Expression (5) is defined as Process 3.

T($\lambda$): transmittance of the topical skin preparation 20 with the sunscreen effect to UV light with the wavelength $\lambda$

S($\lambda$): percentage of the incident light with the wavelength $\lambda$ reflected/scattered on the surface of the topical skin preparation 20 with the sunscreen effect

R($\lambda$): reflectance (= (intensity of the reflection light with the wavelength $\lambda$ detected in Process 2)/(intensity of the incident light with the wavelength $\lambda$ radiated in Process 1))

R$_S$($\lambda$): percentage of the incident light with the wavelength $\lambda$ reflected on the living body

$\beta$($\lambda$): percentage of the incident light with the wavelength $\lambda$ reflected on the interface between the living body and the topical skin preparation 20 with the sunscreen effect

$\gamma$($\lambda$): correction parameter of the transmittance at the wavelength $\lambda$

In the present embodiment, the transmittance T($\lambda$) of the topical skin preparation 20 with the sunscreen effect is calculated by using the measurement value with a model shown in Fig. 4 on the basis of Expression (5) or the like. Then, an SPF value is calculated as a UV protection value on the basis of the calculated transmittance T(λ) as follows. It should be noted that the UV protection value calculated (estimated) by this method will be also referred to as an "estimated UV protection value" or "estimated SPF value".

The transmittance T(λ) can be obtained for one or more wavelengths λ using Expression (5) above and the SPF value can be estimated using Expression (6) below. As the wavelength λ to obtain the transmittance T(λ) that is used when estimating the SPF value, to enhance the estimation accuracy of the SPF value for a formulation using both the UV absorbing agents and the UV scattering agents, out of the wavelengths of 280 nm or more, 400 nm or less, one or two or more wavelengths selected from wavelengths of 290 nm or more, 360 nm or less are favorable, one or two or more wavelengths selected from wavelengths of 290 nm or more, 330 nm or less are more favorable, and one or two or more wavelengths selected from wavelengths of 290 nm or more, 320 nm or less are much more favorable.

[Formula 6]

$$\text{In vitro SPF} = \frac{1}{\sum_{i}^{N} a_i T(\lambda_i) + C}$$

... Expression (6)

N: number of measurement wavelengths
i: index of measurement wavelength (1, 2, ..., N)
$a_i$: multiplication coefficient
C: constant term

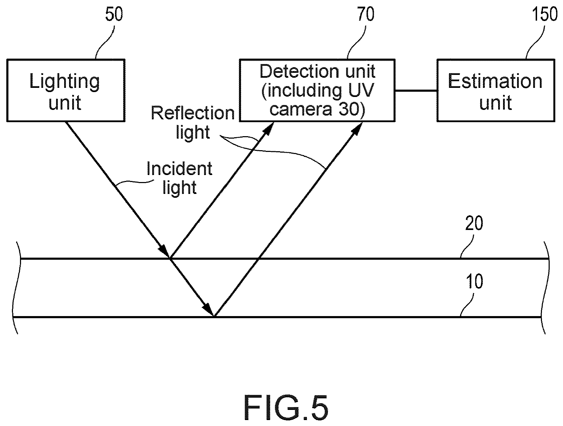

Fig. 5 shows functional blocks (outline) related to estimation of the UV protection value in the present embodiment. The lighting unit 50, the detection unit 70, and the estimation unit 150 described above estimate the UV protection value. It should be noted that Fig. 5 shows functions related to estimation of the UV protection value. Therefore, in reality, the obtaining unit 130 is present between the detection unit 70 and the estimation unit 150. In the present embodiment, an SPF value is estimated as a UV protection value. However, another UV protection value may be estimated.

As shown in Fig. 5, the skin of the user 10 to which the topical skin preparation 20 with the sunscreen effect is applied is irradiated with UV light (incident light) from the lighting unit 50 and its reflection light is detected by the detection unit 70. The UV light (incident light) includes one reflected on the surface of the topical skin preparation 20 with the sunscreen effect, reflection light reflected on the interface to the skin of the user without the UV light (incident light) being absorbed by the topical skin preparation 20 with the sunscreen effect, and reflection light scattered and reflected inside the skin of the user without the UV light (incident light) being absorbed by the topical skin preparation 20 with the sunscreen effect. The UV light (incident light), the reflection light reflected on the surface of the topical skin preparation 20 with the sunscreen effect, the reflection light reflected on the interface between the skin of the user and the topical skin preparation 20 with the sunscreen effect, and the reflection light scattered and reflected inside the skin of the user without the UV light (incident light) being absorbed by the topical skin preparation 20 with the sunscreen effect are detected. Then, the transmittance of the topical skin preparation 20 with the sunscreen effect to UV light is calculated on the basis of Expression (5) above.

Using the transmittance to UV light estimated on the basis of Expression (5), it is sufficient to determine a UV protection effect and uniformity of a coating film formed on the skin of the user by the topical skin preparation 20 with the sunscreen effect and evaluate suitability of the topical skin preparation 20 with the sunscreen effect for the skin of the user. An evaluation method using the uniformity of the coating film formed on the skin of the user by the topical skin preparation 20 with the sunscreen effect will be described later.

On the basis of the transmittance to UV light estimated in the above-mentioned manner and Expression (6), an SPF value is estimated as a UV protection value of the topical skin preparation 20 with the sunscreen effect.

Here, the lighting unit 50 is provided with a light source, a band pass filter, and a polarization plate. The light source emits UV light. The band pass filter allows light in a predetermined wavelength band out of light from the light source to pass therethrough. The polarization plate allows polarized light polarized in a specific direction to pass therethrough. The lighting unit 50 uses light that has passed through them as incident light. With this configuration, it is possible to reliably irradiate the skin of the user 10 with only UV light in a predetermined band wished to be observed (to estimate whether protection is occurring). This can enhance the evaluation accuracy of the estimated UV protection value.

Moreover, the detection unit 70 is provided with the UV camera 30, a band pass filter, and a polarization plate. The band pass filter allows light in a predetermined wavelength band out of reflection light to pass therethrough. The polarization plate allows polarized light polarized in a specific direction to pass therethrough. The reflection light that has passed through the polarization plate and the band pass filter is captured by the UV camera 30. With this configuration, it is possible to detect UV light in a predetermined band wished to be observed (to estimate whether protection is occurring). This can enhance the evaluation accuracy of the estimated UV protection value.

Embodiments 1 to 4 show results of applying topical skin preparations 20 with multiple kinds of sunscreen effects to a plurality of subjects and estimating UV protection values by the above-mentioned method.

<Embodiment 1>

Subject: 7
Application area: 16 cm$^2$ for each area
Formulations: four kinds, A, D, E, and F

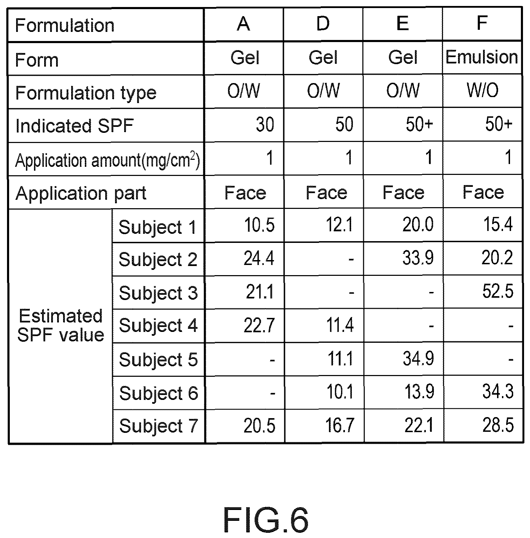

The estimation results are shown in Fig. 6. Fig. 6 shows the estimated SPF values on the basis of the above-mentioned estimation method. It should be noted that a form of each formulation, a formulation type, an indicated SPF value (also referred to as "published SPF value" or "published UV protection value"), an application amount, and an application part are as shown in Fig. 6.

As it is clear from Fig. 6, for the same formulation such as Formulation A, the estimated SPF values differed by more than twofold between Subject 1 and Subject 2. Moreover, for a specific subject, such as Subject 7, Formulation A with an indicated SPF value of 30 yielded an estimated SPF value of 20.5 while a Formulation D with a higher indicated SPF value than Formulation A yielded a lower estimated SPF value of 16.7.

Thus, the estimated SPF values showed variation regardless of the formulation type or subject. Moreover, a formulation with a lower indicated SPF value showed a higher estimated SPF value than a formulation with a higher indicated SPF value in some cases. Therefore, for example, it can be said that there is a possibility that a higher SPF value can be estimated, i.e., the subject can obtain a higher UV protection effect in a case where the subject selects a formulation with a lower indicated SPF value than in a case where the subject selects a formulation with a higher indicated SPF, expecting a higher effect.

As described above, the topical skin preparation 20 with the sunscreen effect generally has a published UV protection value (corresponding to the indicated SPF). Therefore, it is favorable to evaluate suitability by comparing the estimated UV protection value with the published UV protection value.

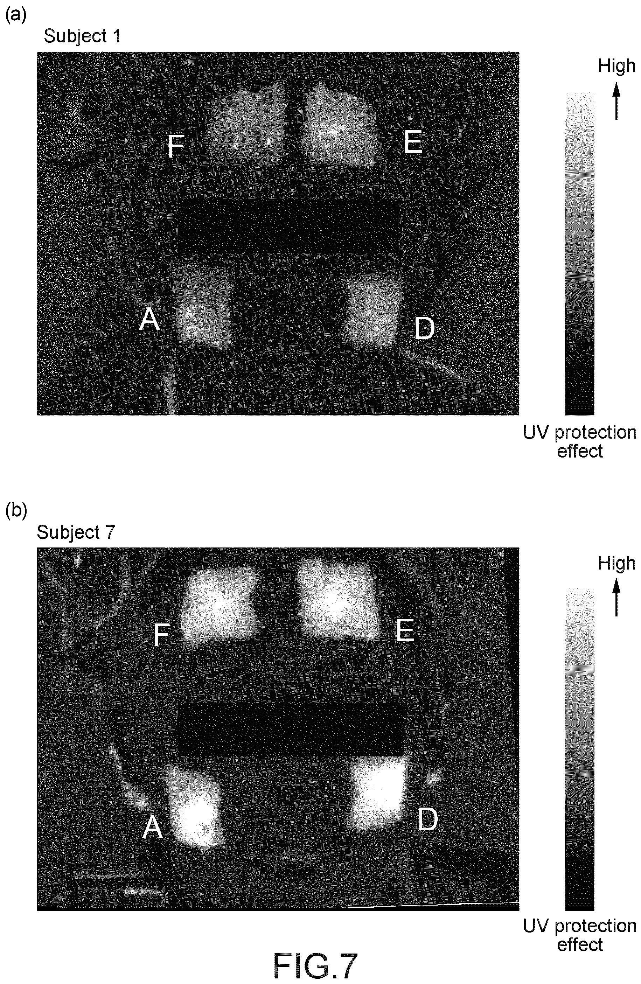

Fig. 7 (a) shows a UV skin image of Subject 1 according to Embodiment 1. Fig. 7 (b) shows a UV skin image of Subject 7 according to Embodiment 1.

Subject 7 shows colors representing higher UV protection values than Subject 1 for all formulations. Thus, displaying not only by the numeric values as in Fig. 6, but also by the images as in Fig. 7, makes it possible to visually understand which formulation is suitable. That is, for example, using the display in Fig. 7 (a) and (b) makes it possible to evaluate the suitability of the topical skin preparation 20 with the sunscreen effect for the skin of the user on the basis of reflection light (UV skin images) without estimating UV protection values. In this case, the evaluation becomes possible by preparing a reference UV skin image.

<Embodiment 2>

Subject: 7
Application area: 16 cm$^2$ for each area
Formulations: four kinds, A, D, E, and F

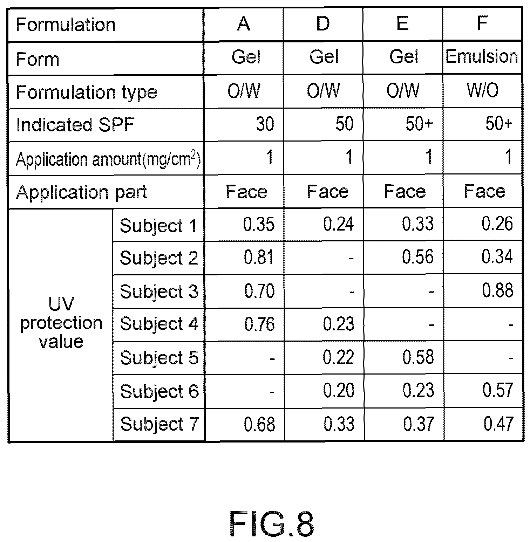

Estimation results are shown in Fig. 8. The UV protection values shown in Fig. 8 are values obtained by dividing the estimated SPF values by the indicated SPF values on the basis of the above-mentioned estimation method. It should be noted that a form of each formulation, a formulation type, an indicated SPF value (published SPF value), an application amount, and an application part are as shown in Fig. 8.

The indicated SPF (value) of each of Formulation E and Formulation F was 50+. Therefore, for the sake of convenience, provided that the indicated SPF value was 60, calculation was performed. It is because many commercially available products with an indicated SPF value of 50+ often have an SPF value of about 60.

When the UV protection value shown in Fig. 8 is greater than 1, the estimated SPF value is greater than the indicated SPF value. Moreover, as the UV protection value is closer to 1, the estimated SPF value is closer to the indicated SPF value. Therefore, one greater than 1 or closer to 1 can be evaluated as a formulation suitable for the subject's skin.

Therefore, it becomes easy to know which formulation is suitable. It should be noted that in Embodiment 2, the application amount is 1 mg/cm$^2$, so the estimated SPF value is typically less than or equal to the indicated SPF value (indicated SPF is measured under the condition that the application amount is 2 mg/cm$^2$). Therefore, the UV protection value (divided value) is assumed to be less than 1. Therefore, as it is closer to 1, it can be determined to be more suitable.

It should be noted that the minimum dose of radiation that produces skin erythema of the subject is called minimal erythema dose (MED). The indicated SPF is calculated as a value of MED of the subject's skin to which the sunscreen is applied with respect to MED of the subject's bare skin to which the sunscreen is applied.

The indicated SPF is an average of measurement values of several subjects. On the other hand, with the estimation approach according to the present embodiment, it is possible to know a UV protection effect for the user him or herself.

For example, some formulation contain additives, such as ultraviolet protective agents and film-forming agents, in order to increase the indicated SPF. Such components can cause burden on the skin. In this regard, if the user can select a formulation, which has a low indicated SPF, but has a high UV protection effect for the user him or herself, it would be possible to reduce such burden.

Moreover, some users prefer formulations with a low indicated SPF for preferences and the like other than the above-mentioned reason. Therefore, it is possible to notify such users of formulations whose UV protection effects for the users are high even through the indicated SPFs are low.

It should be noted that also with respect to a formulation whose indicated SPF is not published, the information processing apparatus 100 may calculate an indicated SPF by the above-mentioned calculation approach targeting a plurality of other subjects for which the above-mentioned MED before and after sunscreen application has been measured, and calculate a UV protection value by dividing an SPF value estimated for a target user on the basis of the above-mentioned estimation method by that indicated SPF. In the present embodiment and the following embodiments, it is assumed that the "indicated SPF" also includes an indicated SPF calculated by this information processing apparatus 100.

<Embodiment 3>

Subject: 5
Application area: 16 cm$^2$ for each area
Formulations: four kinds, P, Q, R, and S, the four kinds all have the same indicated SPF value of 50+

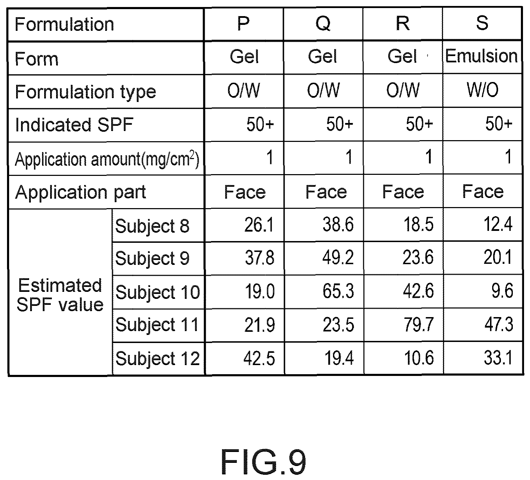

Estimation results are shown in Fig. 9. Fig. 9 shows estimated SPF values on the basis of the above-mentioned estimation method. It should be noted that a form of each formulation, a formulation type, an indicated SPF value (published SPF value), an application amount, and an application part are as shown in Fig. 9.

As it is clear from Fig. 9, even with the same indicated SPF value, the estimated SPF value varies depending on the subject. Moreover, for Subjects 8 to 10, Formulation Q is a formulation whose estimated SPF value is closer to the indicated SPF value. For Subject 11, Formulation R is a formulation whose estimated SPF value is closer to the indicated SPF value. For Subject 12, Formulation P is a formulation whose estimated SPF value is closer to the indicated SPF value. In this manner, the formulation whose estimated SPF value is closer to the indicated SPF value differs depending on the subject. Therefore, in particular, it is useful and favorable to simultaneously compare formulations with the same indicated SPF value on the same face for proposing a formulation suitable for the user. In Embodiment 3, all formulations have the same indicated SPF value of 50+, but their estimated SPF values differ depending on the subject. Thus, it is possible to propose a topical skin preparation with a sunscreen effect with the highest suitability for that subject.

In this manner, topical skin preparations with multiple kinds of sunscreen effects that have published UV protection values (corresponding to the indicated SPF values) are applied to the skin of the user. Then, the reflection light of UV light radiated to the skin is obtained (UV skin image is obtained by imaging the skin of the user). In such a case, since at least two or more of the published UV protection values of the topical skin preparations with multiple kinds of sunscreen effects are the same (Formulations P, Q, R, and S all have an indicated SPF value of 50+), a topical skin preparation 20 with a sunscreen effect whose estimated SPF value is the closest to the published UV protection value can be determined to have high suitability for a user expecting a higher UV protection effect. Therefore, a proposal useful for the user can be performed.

<Embodiment 4>

Subject: 4 (A, B, C),
3 (D, E, F)
Application area: 16 cm$^2$ for each area
Formulations: six kinds, A, B, C, D, E, and F, the six kinds all have an indicated SPF value of 15 to 32. Moreover, in Embodiment 4, a twofold amount of the topical skin preparation 20 with the sunscreen effect was applied (to match measurement conditions of the indicated SPF value) as compared to Embodiments 1 to 3.

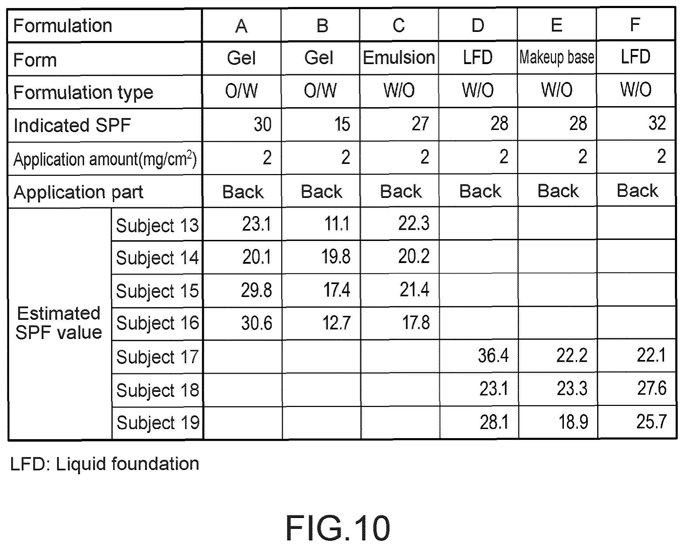

Estimation results are shown in Fig. 10. Fig. 10 shows estimated SPF values on the basis of the above-mentioned estimation method. It should be noted that a form of each formulation, a formulation type, an indicated SPF value (published SPF value), an application amount, and an application part are as shown in Fig. 10.

As it is clear from Fig. 10, as in Embodiment 3, a difference between the estimated SPF value for each subject and the indicated SPF value differs depending on the formulation (some estimated SPF values exceed the corresponding indicated SPF values). A formulation whose estimated SPF value exceeds the indicated SPF value is the most suitable formulation for the subject's skin. In a case where the estimated SPF value is smaller than the indicated SPF value, a formulation closer to the indicated SPF value is a formulation suitable for that subject.

Here, in Embodiment 3, those with the same indicated SPF value are topical skin preparations 20 with sunscreen effects as evaluation targets, though not limited thereto. For example, those in the same form may be the topical skin preparations 20 with the sunscreen effects as the evaluation targets. In Embodiment 3, the indicated SPF values of the topical skin preparations 20 with the sunscreen effects as the evaluation targets are 40 or more. In Embodiment 4, the indicated SPF values of the topical skin preparations 20 with the sunscreen effects as the evaluation targets are less than 40, though not limited thereto. For example, the indicated SPF values of the topical skin preparations 20 with the sunscreen effects as the evaluation targets may be 30 or more or may be less than 30. Alternatively, for example, the indicated SPF values of the topical skin preparations 20 with the sunscreen effects as the evaluation targets may be 20 or more or may be less than 20. Moreover, for example, topical skin preparations 20 with sunscreen effects included in a specific group roughly classified into purposes, a daily low SPF value (SPF value of 20 or less), a medium SPF value (around SPF 30), and a leisure SPF value (SPF 50, 50+) may be the topical skin preparations 20 with the sunscreen effects as the evaluation targets. Moreover, rather than the indicated SPF value, for example, the topical skin preparations 20 with the sunscreen effects as the evaluation targets may be those with an indication, "low irritation" or "for sensitive skin," or those with no indication, "low irritation" or "for sensitive skin." Alternatively, the topical skin preparations 20 with the sunscreen effects as the evaluation targets may be those with an indication, "for children" or "for kids," or those with no indication, "for children" or "for kids." Alternatively, for example, the topical skin preparations 20 with the sunscreen effects as the evaluation targets may be non-chemical ones or chemical ones. Alternatively, for example, the topical skin preparations 20 with the sunscreen effects as the evaluation targets may be those not containing specific components (e.g., alcohol, preservative, and the like) or those containing specific components (e.g., alcohol, preservative, and the like). That is, the topical skin preparations 20 with the sunscreen effects as the evaluation targets may be those including a common matter, such as a UV protection value, components contained in the topical skin preparations 20 with the sunscreen effects, a function of the topical skin preparations 20 with the sunscreen effects, or a purpose of the topical skin preparations 20 with the sunscreen effects. With this configuration, it is possible for the user to know a topical skin preparation 20 with a sunscreen effect optimal for the user from those common to the published information of the topical skin preparations 20 with the sunscreen effects. It is possible to provide more useful information for the user.

As shown in Embodiments 1 to 4, even for the same topical skin preparation 20 with a sunscreen effect, the estimated SPF value varied depending on the subject. Moreover, even for the same subject, the estimated SPF value was close to the indicated SPF value (including cases where the estimated SPF value was higher than the indicated SPF value) or not, depending on the topical skin preparation 20 with the sunscreen effect, and no consistent trend was observed. Therefore, using this estimation method to estimate the SPF values of topical skin preparations 20 with multiple kinds of sunscreen effects can be useful for proposing a topical skin preparation 20 with a sunscreen effect optimal for the user.

In this manner, by obtaining reflection light of UV light radiated to the skin of the user to which topical skin preparations 20 with multiple kinds of sunscreen effects are applied (imaging the skin of the user and obtaining the UV skin image), estimating a UV protection value for each of the topical skin preparations 20 with the sunscreen effects, and extracting a topical skin preparation 20 with a sunscreen effect that has high suitability by using the estimated UV protection value (e.g., extracting a sunscreen topical skin preparation 20 with a high estimated SPF value), it is possible to propose a topical skin preparation 20 with a sunscreen effect that has a high UV protection effect for the user.

Here, in general, the user often expects the topical skin preparation 20 with the sunscreen effect to provide not only the UV protection effect, but also whether the topical skin preparation 20 with the sunscreen effect can be applied to the skin uniformly (whether a uniform coating film of the topical skin preparation 20 with the sunscreen effect is formed on the skin), a use experience (application feeling), application easiness, and the like. In view of this, in the present embodiment, it is also possible to evaluate the suitability of the topical skin preparation 20 with the sunscreen effect for the skin of the user not only for the UV protection effect, but also for the use experience, the application easiness, and the like by using the uniformity of the coating film of the topical skin preparation 20 with the sunscreen effect and questions on the basis of the estimated UV protection value and determine suitability.

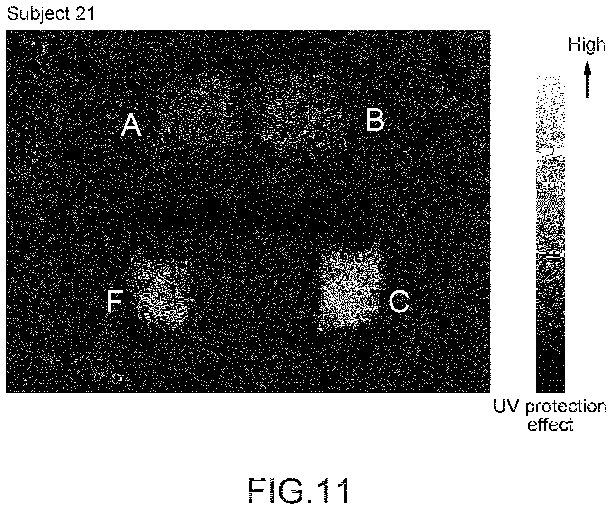

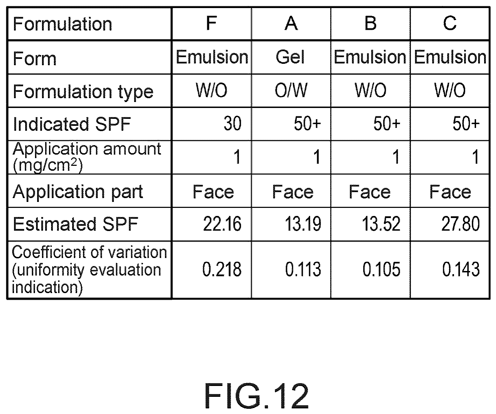

An estimation method for the uniformity of the coating film formed by the topical skin preparation 20 with the sunscreen effect will be described with reference to Figs. 11 and 12.

Fig. 11 is a UV skin image of Subject 21 for which Formulation A was applied to the right forehead, Formulation B was applied to the left forehead, Formulation F was applied to the right cheek, and Formulation C was applied to the left cheek.

First of all, a UV protection value is estimated for each pixel of an evaluation region from reflection light of UV light radiated to this Subject 21 (the estimation method for the UV protection value is an estimation method described in <Estimation Method for UV Protection Value>). Next, a coefficient of variation is determined from the estimated UV protection value for each pixel as a uniformity evaluation indication of a coating film formed in the evaluation region. It can be said that the uniformity of the coating film formed in the evaluation region is higher as the coefficient of variation is lower.

Fig. 12 shows a form of each formulation applied to Subject 21 in Fig. 11, the formulation type, the indicated SPF value (published SPF value), the application amount, the application parts, the estimated UV protection value (SPF value), and a calculated uniformity evaluation indication (coefficient of variation).

As shown in Fig. 12, the uniformity evaluation indication (coefficient of variation) of Formulation C is low. Therefore, it can be said that the uniformity of the coating film formed in the evaluation region is high. In addition, Formulation C can be said to be a formulation with the highest suitability for the skin of Subject 21 because the estimated UV protection value is a value higher than Formulation A, Formulation B, and Formulation F.

In this manner, Formulation C and Formulation F may be evaluated to have substantially the same suitability for the skin of Subject 21 only as to the estimated UV protection value. However, when the uniformity of the coating film formed in the evaluation region is added as a determination element, it is possible to extract a formulation (in this case, Formulation C) having much higher suitability.

Next, a method of using the use experience, the application easiness, and the like of the user with respect to the topical skin preparation 20 with the sunscreen effect for evaluation will be described.

The application easiness when applying the topical skin preparation 20 with the sunscreen effect, irritating on the skin after application (e.g., whether there is any tingling or tightness), and the like are included in questions (questionnaire) as items related to the use experience. The user's answer result is used for the evaluation. When the user selects the topical skin preparation 20 with the sunscreen effect, the evaluation related to the user's use experience is important. Therefore, it is favorable to include the evaluation related to the user's use experience in the evaluation contents.

Moreover, items that the user prioritizes when selecting the topical skin preparation 20 with the sunscreen effect, other than the user's use experience and the application easiness, are included in the questions (questionnaire) (for example, the user prefers to prioritize the UV protection effect rather than the use experience or the user prefers to prioritize not only the UV protection effect, but also other effects (e.g., tone-up effect, moisturizing effect)) so that the user can answer what the user seeks in the topical skin preparation 20 with the sunscreen effect. In this manner, these matters may be included in the evaluation.

In this manner, items other than the estimated UV protection value are also included in the suitability of the topical skin preparation 20 with the sunscreen effect for the skin of the user. This enables recommendation of an optimal sunscreen topical skin preparation 20 to the user. For example, it is possible to extract one with high suitability from formulations having substantially the same indicated SPF value and estimated SPF value by using a use experience and effects other than the UV protection effect that are preferred by the user as criteria to determine suitability. Moreover, it is possible to extract one with high suitability from formulations common to a use experience and effects other than the UV protection effect that are preferred by the user on the basis of the estimated SPF value.

Hereinafter, an example of evaluation flow is shown.

1) The user answers a relevant questionnaire before applying a formulation. An example of the questionnaire is shown below.

- Skin type (whether or not the user has sensitive skin)
- Skin reaction to UV (e.g., the skin tends to turn red or darken easily)
- Whether the user prioritizes either the UV protection effect or the use experience
- Minimal SPF value (which SPF value the user seeks at least)
- Age (whether or not the user is a kid)
- Main use scene (for daily or leisure use)
- Main use site (face, body, or face and body)

2) Estimate a UV protection value and uniformity of the coating film formed by the topical skin preparation 20 with the sunscreen effect on the basis of a UV skin image

- Estimate UV protection values of topical skin preparations 20 with multiple kinds of sunscreen effects by the estimation method described in <Estimation Method for UV Protection Value>. The UV protection values to be estimated are favorably indications (in the present embodiment, SPF values) indicated on the topical skin preparation 20 with the sunscreen effect.
- Also estimate uniformity of a coating film of a formulation formed by each of the topical skin preparations 20 with the sunscreen effects

3) The user answers a questionnaire after applying the formulation. An example of the questionnaire is shown below.

- How is the application feel? (Do you apply it easily without streaks?)
- Do you feel irritating on the skin?
- Do you feel moisturizing or dry?
- Do you feel smooth or sticky?
- How good is the finish?
- Does it blurs pores?
- How well does makeup apply over it?

By configuring to answer the questionnaire also after the application in this manner, application easiness and application feeling when the user applying it, the presence/absence of streaks, and the like can be included in the suitability evaluation indication. Moreover, in general, one that the user finds easy to apply or one that causes no streaks is a formulation whose coating film is easily generated or a topical skin preparation 20 with a sunscreen effect that the user can easily uniformly apply. Therefore, the suitability for the skin can be evaluated by answering the questionnaire.

In a case where the user wishes to prioritize the UV protection effect on the basis of "Whether the user prioritizes either the UV protection effect or the use experience" in 1), the sunscreen topical skin preparation 20 for which the estimated SPF value is high is recommended. Alternatively, with the same estimated SPF value, for example, the topical skin preparation 20 with the sunscreen effect for which the indicated SPF value is low is recommended in a case where the user has sensitive skin (because in general, the topical skin preparation with the sunscreen effect for which the indicated SPF value is lower has a lower blend ratio of UV absorbing agents, so the degree of irritation felt by the user can be considered lower). Otherwise, in a case where the user does not have sensitive skin, the topical skin preparation 20 with the sunscreen effect for which the application feel is good is recommended.

With this configuration, at least one of the estimated SPF value and uniformity of the coating film of the topical skin preparation 20 with the sunscreen effect created on the skin of the user and easiness for creation of the coating film of the topical skin preparation 20 with the sunscreen effect on the skin of the user can be included in the suitability. Moreover, the suitability of the topical skin preparation 20 with the sunscreen effect for the skin of the user can be evaluated from various viewpoints by using the UV protection value. Therefore, it is possible to propose a topical skin preparation 20 with a sunscreen effect optimal for the user.

In this manner, the user answers a predetermined questionnaire and suitability of the topical skin preparation with the sunscreen effect for the skin of the user evaluated by using the estimated UV protection value and the answer. Accordingly, it is possible to propose to the user a topical skin preparation 20 with an optimal sunscreen effect considering the UV protection effect and the other information.

It should be noted that the suitability evaluation based on the above-mentioned flow may automatically propose the topical skin preparation 20 with the sunscreen effect for which the suitability is high (or low) on the basis of an evaluation program generated in advance or may propose the topical skin preparation 20 with the sunscreen effect for which the suitability is high (or low) on the basis of a flowchart generated in advance.

Moreover, when proposing a topical skin preparation 20 with a sunscreen effect optimal for the user, only one optimal formulation is proposed or a plurality of top formulations is proposed. In a case of prioritizing the UV protection effect, formulations are proposed in descending order (or ascending order) of the estimated SPF value or topical skin preparations 20 with the sunscreen effect for which the estimated SPF value is greater than or equal to a predetermined value (or less than or equal to the predetermined value) are proposed, for example. Any proposal can be thus performed. It is sufficient to determine the proposal contents in consideration of demands from the user. Moreover, even in a case where the user wishes to prioritize the UV protection effect for example, the topical skin preparation 20 with the sunscreen effect for which the estimated SPF value is high is sometimes inappropriate from the perspective of the questionnaire and the uniformity of the coating film formed by the topical skin preparation 20 with the sunscreen effect. In such a case, a recommended topical skin preparation 20 with a sunscreen effect may be proposed together with a desired topical skin preparation 20 with a sunscreen effect.

<Evaluation System>

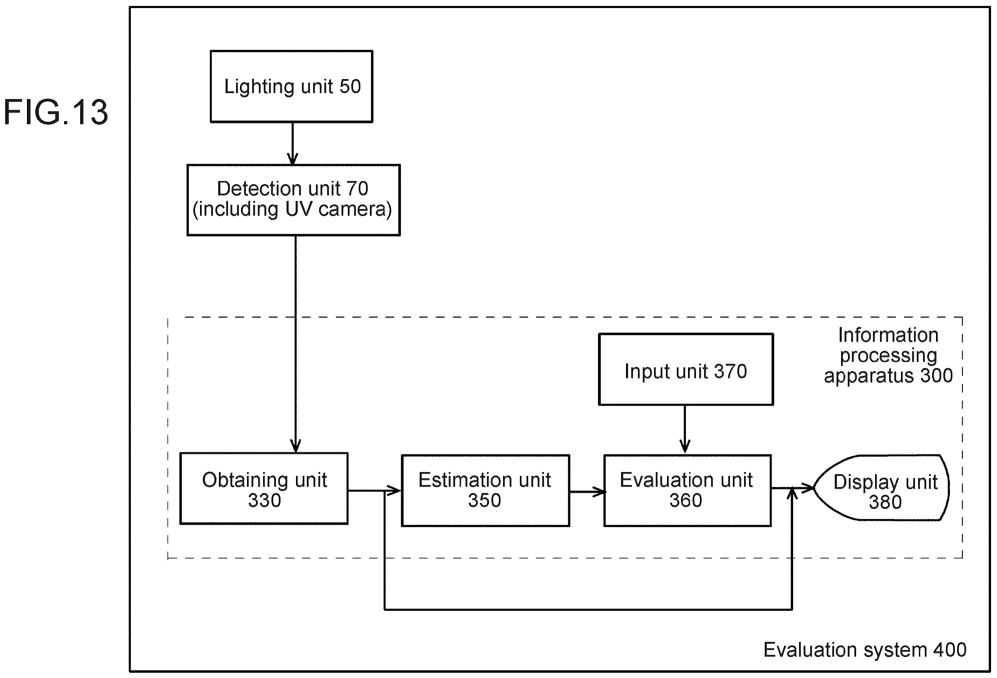

An evaluation system 400 will be described with reference to Fig. 13.

The evaluation system 400 in the present embodiment is constituted by an obtaining unit 330, an evaluation unit 360, a lighting unit 50, and a detection unit 70. The evaluation system 400 includes an information processing apparatus 300 capable of executing various types of processing. The obtaining unit 330 and the evaluation unit 360 are provided in the information processing apparatus 300.

The information processing apparatus 300 is a general-purpose personal computer. The information processing apparatus 300 includes an input unit 370 such as a keyboard, a touch panel, and a pointing device, an arithmetic processing unit (e.g., a CPU or the like), a storage unit, an input/output interface, and the like.

The obtaining unit 330 is a means (obtaining means) for irradiating the skin of the user to which the topical skin preparation with the sunscreen effect is applied with UV light and obtaining reflection light of the irradiated UV light. The obtaining unit 330 obtains it from the detection unit 70 via a network, for example.

The evaluation unit 360 is a means (evaluation means) for evaluating the suitability of the topical skin preparation 20 with the sunscreen effect for the skin of the user by displaying data (e.g., UV skin image) obtained by the obtaining unit 330 on a display unit 380 or using the UV protection value estimated by an estimation unit 350. An evaluation result by the evaluation unit 360 is favorably adjusted to have a display format so that an evaluator can easily understand it through the display unit 380. It should be noted that the "evaluator" set forth herein includes both a store clerk and a user.

Moreover, the information processing apparatus 300 favorably includes the estimation unit 350, the input unit 370, and the display unit 380 (display device). However, the display unit 380 may be provided outside the information processing apparatus 300 and connected via a network or the like.

The estimation unit 350 is a means for irradiating the skin of the user to which the obtained topical skin preparation with the sunscreen effect is applied with UV light and estimating a UV protection value from reflection light of the irradiated UV light.

The display unit 380 is a means for irradiating the skin of the user to which the obtained topical skin preparation with the sunscreen effect is applied with UV light and displaying intensity of the reflection light, display of the UV skin image, an estimation result of estimation processing, and an evaluation result of evaluation processing, or the like on the basis of the reflection light of the irradiated UV light. It should be noted that it is also a means for displaying the contents of questions to the user.

Moreover, the lighting unit 50 (irradiation means) that irradiates the skin of the user to which the topical skin preparation with the sunscreen effect is applied with UV light and the detection unit 70 (detection means) is necessary. It is assumed that the detection unit 70 includes the above-mentioned UV camera 30. It should be noted that the lighting unit 50 and the detection unit 70 are similar to those described above, so the description will be omitted.

The storage unit of the information processing apparatus 300 has stored a program for executing the above-mentioned evaluation method. The skin of the user to which the topical skin preparation 20 with the sunscreen effect is applied, which is obtained by the obtaining unit 330, is irradiated with UV light. Using the reflection light of the irradiated UV light, the program causes the evaluation unit 360 to evaluate the suitability of the topical skin preparation 20 with the sunscreen effect for the skin of the user.

<Application Program>

The evaluation apparatus 200 has installed an application program (hereinafter, also referred to as this program) for causing the evaluation apparatus 200 to execute the above-mentioned evaluation method.

Similarly, the information processing apparatus 300 has installed an application program (hereinafter, also referred to as this program) for causing the information processing apparatus 300 to execute the above-mentioned estimation method. In the present embodiment, the application program is application software that irradiates the skin of the user to which the topical skin preparation 20 with the sunscreen effect is applied with UV light and evaluates suitability of the topical skin preparation with the sunscreen effect for the skin of the user by using the reflection light of the irradiated UV light. Such a program can be realized also as a program product.

This program includes obtaining processing and evaluation processing.

The obtaining processing is processing of irradiating the skin of the user to which the topical skin preparation with the sunscreen effect is applied with UV light and obtaining reflection light of the irradiated UV light. It should be noted that in the present embodiment, data based on the reflection light of the irradiated UV light is obtained, and calculation of the intensity of the reflection light of the UV light and generation of the UV skin image are performed in the obtaining processing. However, for example, calculation of the intensity of the reflection light of the UV light and generation of the UV skin image may be performed by an apparatus (not shown) different from the evaluation apparatus 200, and the calculated intensity of the reflection light of the UV light and the generated UV skin image may be obtained.

The evaluation processing is processing of evaluating the suitability of the topical skin preparation 20 with the sunscreen effect for the skin of the user by using reflection light.

Moreover, this program favorably further includes evaluation processing and display processing.

The estimation processing is processing of irradiating the skin of the user to which the obtained topical skin preparation with the sunscreen effect is applied with UV light and estimating the UV protection value from the reflection light of the irradiated UV light.

The display processing is processing of irradiating the skin of the user to which the obtained topical skin preparation with the sunscreen effect is applied with UV light and displaying, on the basis of the reflection light of the irradiated UV light, display of intensity of the reflection light and the UV skin image, an estimation result of estimation processing, and an evaluation result by the evaluation processing.

As described above, the present invention has been described showing the specific embodiments. However, the present invention is not limited to the above-mentioned embodiments. The present invention also includes aspects such as various variants and improvements as long as the objects of the present invention can be accomplished.

<Modified Examples>

In the present embodiment, when estimating the SPF value as the UV protection value, a multispectral image (intensity of light with each wavelength) is captured and output, a spectrum of the transmittance of the topical skin preparation 20 with the sunscreen effect is calculated by using the multispectral image, and an SPF value is estimated on the basis of Expression above (6), though not limited thereto. For example, the detection unit 70 may detect and output the intensity of light of narrow-band wavelengths of a measurement target and estimate the SPF value on the basis of the transmittance of the topical skin preparation 20 with the sunscreen effect to UV light with the narrow-band wavelengths and Expression (7) shown below.

[Formula 7]

$$\mathrm{SPF} = \frac{1}{aT(\lambda)}$$

$$\dots \text{Expression (7)}$$

a: multiplication coefficient of T($\lambda$)

Where the "narrow-band wavelengths" are a narrow wavelength band of 15 nm or less. It may be a single wavelength or may be a wavelength band with a width of 10 nm. It should be noted that in this case, the lighting unit 50 is assumed to be capable of radiating UV light with a short-band wavelength (290 nm or more, less than 325 nm). Moreover, the lighting unit 50 may include a polarization plate and an optical filter or does not need to include them. Moreover, the configuration of the detection unit 70 is similar to that described above. It is sufficient to use the UV camera 30 with performance according to the method of estimating the UV protection value. Using the multispectral image takes a long time because measurements are performed for every wavelength. On the other hand, wavelengths to be measured are limited in a case of using the narrow-band wavelengths. It can shorten the imaging time, such that the burden on the user can be reduced. It should be noted that in this case, it may be an average of the intensity of reflection light at a plurality of positions in a region of the skin of the user to which the topical skin preparation 20 with the sunscreen effect is applied. It is sufficient to determine whether to determine the SPF value on the basis of Expression (6) or Expression (7) in consideration of the performance of devices provided in the lighting unit 50 and the detection unit 70. Regardless of which of these methods is adopted, it is possible to evaluate the suitability of the topical skin preparation 20 with the sunscreen effect for the skin of the user.

In the present embodiment, Fig. 7 (a) and (b) are used as the example of evaluating the suitability of the topical skin preparation 20 with the sunscreen effect for the skin of the user on the basis of reflection light without estimating the UV protection value, though not limited thereto. For example, it is possible to evaluate the suitability of the topical skin preparation 20 with the sunscreen effect for the skin of the user even by using the intensity (numeric value) of the reflection light. It should be noted that with the UV skin image as in Fig. 7 (a) and (b) and the estimated UV protection value and the like, the user can more easily understand the degree of UV protection of a topical skin preparation 20 with a sunscreen effect as a target. Therefore, it is more favorable to also use the UV skin image.

In all Embodiments 1 to 4, the suitability is evaluated in a case where the application amounts of the topical skin preparations 20 with multiple kinds of sunscreen effects are the same, though not limited thereto. For example, all application areas for Formulation A in the embodiment may be set to 16 cm$^2$, a pattern to apply 8 mg with respect to the bare skin, a pattern to apply 16 mg with respect to the bare skin, a pattern to apply 24 mg with respect to the bare skin, a pattern to apply 32 mg with respect to the bare skin may be set, and each UV protection value may be estimated. With this configuration, it is possible to understand the application amount by which the UV protection effect can be sufficiently provided. It should be noted that the UV protection effect does not change beyond a predetermined application amount. Therefore, it is possible to present a suitable application amount to the user.

It should be noted that face washing was performed for each application pattern and a predetermined amount was applied to the bare skin with the same area. However, for example, different application amounts, for example, 8 mg for the right cheek, 16 mg for the left cheek, 24 mg for the left forehead, and 32 mg for the right forehead, may be applied to the bare skin with the same area and four patterns of UV skin images of the skin of the user may be simultaneously captured. With this configuration, there are an advantage of reducing an impact of a change of the skin due to an environment at the time of measurement and an advantage of shortening the time required to make a proposal. Then, also with this configuration, it is possible to understand an application amount with a high UV protection effect.

Moreover, in Embodiments 1 to 3, the application amount of the topical skin preparation 20 with the sunscreen effect is set to 1 mg/1 cm$^2$ and in Embodiment 4, it is set to 2 mg/1 cm$^2$, though not limited thereto. The application amount of the topical skin preparation 20 with the sunscreen effect is favorably 0.5 mg/1 cm$^2$ to 4 mg/1 cm$^2$, and more favorably 1 mg/1 cm$^2$ to 2 mg/1 cm$^2$.

Moreover, in Embodiments 1 to 4, the application area of the topical skin preparation 20 with the sunscreen effect is set to 16 cm$^2$, though not limited thereto. The application area of the topical skin preparation 20 with the sunscreen effect only needs to be 4 cm$^2$ or more, and favorably 9 cm$^2$ or more.

In the present embodiment, the UV skin image may be captured at any timing after the topical skin preparation with the sunscreen effect is applied, though not limited thereto. For example, the UV skin image may be obtained when a different time has elapsed since application, for example, immediately after the same topical skin preparation 20 with a sunscreen effect is applied to the skin of the user, 1 hour later after application, 2 hours later after application, or 3 hours later after application, and the UV protection value may be estimated. Moreover, for example, a percentage of each of the estimated UV protection values 1 hour later after application, 2 hours later after application, and 3 hours later after application to the estimated UV protection value immediately after application may be calculated. With this configuration, it is possible to evaluate suitability of the estimated UV protection value corresponding to an elapse time since application.

In the present embodiment, the UV skin image is captured in the state in which the topical skin preparation 20 with the sunscreen effect is applied, though not limited thereto. For example, a UV skin image may be captured after the topical skin preparation 20 with the sunscreen effect is applied, and then a UV skin image may be captured again and a UV protection value may be respectively estimated, for example, after some workout is done for a predetermined time or after staying in a hot and humid room for a predetermined time. With this configuration, it is possible to estimate a UV protection value in a case of sweating in the state in which the topical skin preparation 20 with the sunscreen effect is applied.

In the present embodiment, the UV skin image is captured in a state in which the topical skin preparation 20 with the sunscreen effect is applied, though not limited thereto. For example, a UV skin image may be captured after applying basic skincare products (lotion, emulsion, and the like) and the topical skin preparation 20 with the sunscreen effect after face washing, and then a UV skin image may be captured again and each UV protection value may be estimated after applying makeup (e.g., makeup base, foundation, point makeup). With this configuration, it is possible to estimate a UV protection value in a state in which total care (full makeup) is done after the topical skin preparation 20 with the sunscreen effect is applied.

In the present embodiment, in Embodiment 3, the topical skin preparation 20 with the sunscreen effect that has the indicated SPF value of 40 or more is evaluated. Many users prefer topical skin preparations 20 with sunscreen effects that have a high SPF value. In general, those users are highly interested in the UV protection effects. Therefore, it is useful for them to understand topical skin preparations 20 with sunscreen effects optimal for the users. This is why the topical skin preparation 20 with the sunscreen effect that has the indicated SPF value of 40 or more is evaluated. In Embodiment 3, the indicated SPF values of the four kinds of formulations are set to 50+, though not limited thereto. Topical skin preparations 20 with sunscreen effects that have various values greater than or equal to 40 may be used. Moreover, those users can be highly interested in how much degree they can expect the UV protection effect with respect to the indicated SPF value. Therefore, as in Embodiment 2, a value obtained by dividing the estimated SPF values by the indicated SPF values may be estimated as a UV protection value.

In the present embodiment, in Embodiment 4, the topical skin preparation 20 with the sunscreen effect that has the indicated SPF value less than 40 is evaluated. Many users prefer topical skin preparations 20 with sunscreen effects that have a low indicated SPF value. In general, those users are often sensitive-skin users, users having sensitive skin. Therefore, it is useful for them to understand topical skin preparations 20 with sunscreen effects optimal for the users also considering the use experience. This is why the topical skin preparation 20 with the sunscreen effect that has the indicated SPF value less than 40 is evaluated. In view of this, it is useful to have the user answer the questionnaire and propose a topical skin preparation 20 with a sunscreen effect optimal for the user in consideration of the answer result. Moreover, there is a need for knowing those having a high estimated SPF value among those having a low indicated SPF value (among low-irritation topical skin preparations with sunscreen effects). Therefore, it is also useful to propose the topical skin preparation 20 with the sunscreen effect that has a high estimated SPF value.

In the present embodiment, the evaluation is performed using the topical skin preparation 20 with the sunscreen effect that has an indicated SPF value of 40 or more and the topical skin preparation 20 with the sunscreen effect that has an indicated SPF value of less than 40. In general, it is known that as the way of choosing a cosmetic product for UV protection depending on a life scene, it is favorable to choose one of topical skin preparations with sunscreen effects that have an SPF value of 40 or more for long-time outdoor activity in view of the sunscreen protection effect and it is favorable to choose one of topical skin preparations with sunscreen effects that have an SPF value of less than 40 for daily life and short-time outdoor activity in view of balance between sunscreen effect and use experience. Considering this, a store clerk and a user choose topical skin preparations 20 with sunscreen effects. This is why the evaluation is performed using the topical skin preparation 20 with the sunscreen effect that has an indicated SPF value of 40 or more and the topical skin preparation 20 with the sunscreen effect that has an indicated SPF value of less than 40. In view of this, in the present embodiment, by performing the evaluation using the topical skin preparation 20 with the sunscreen effect that has an indicated SPF value of 40 or more and the topical skin preparation 20 with the sunscreen effect that has an indicated SPF value of less than 40, evaluation assuming a scene where the user uses the topical skin preparation 20 with the sunscreen effect can be performed. Therefore, it is possible to provide a result more useful for the user.

<Additional Example 1>

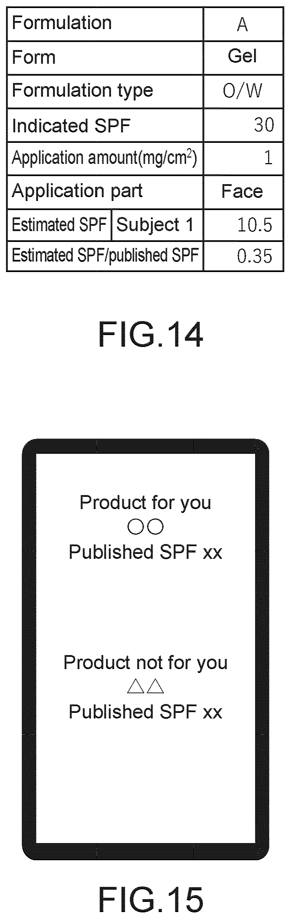

Fig. 14 is a view showing a result of evaluating suitability of a topical skin preparation for the user in another embodiment of the present invention.

As described above, in a case where the estimated SPF value exceeds the indicated SPF value, as the excessive value is larger, it means a formulation more suitable for the skin of the user. In a case where the estimated SPF value is smaller than the indicated SPF value, a formulation close to the indicated SPF value can be said to be a formulation suitable for that user.

Therefore, as the value obtained by subtracting the indicated SPF value from the estimated SPF value is larger or as a ratio of the estimated SPF value to the indicated SPF value is larger, the suitability of the topical skin preparation for the skin of the user is evaluated higher.

Then, the information processing apparatus 100 or 300 sends information that recommends a topical skin preparation evaluated to have high suitability for the skin of the user to the user or information that does not recommend a topical skin preparation evaluated to have low suitability for the skin of the user to the user on the basis of the above-mentioned subtracted value or ratio to the information processing terminal of the user for displaying that information.

In the example of the figure, regarding single Formulation A, a ratio of the estimated SPF value to the indicated SPF value is calculated on the basis of the above-mentioned reflection light.

Then, as shown in Figs. 15 and 14, as to each formulation, on the basis of the ratio of the estimated SPF value to the indicated SPF value, a formulation to be recommended to the user (with high suitability) or a formulation not to be recommended to the user (with low suitability) may be displayed on the information processing terminal of the user together with its indicated SPF value.

In this case, in accordance with the above-mentioned ratio value or value obtained by subtracting the indicated SPF value from the estimated SPF value, a recommendation level to the user may be calculated and displayed as a multi-scale evaluation value (e.g., number of stars), such as "Recommendation level: 2-star rating." Moreover, on the basis of a degree of similarity of the above-mentioned ratio value or subtracted value, information recommending other products, such as "... is also recommended to you," may be displayed.

It should be noted that hereinabove, the suitability of the topical skin preparation for the skin of the user is evaluated on the basis of the estimated SPF value and the indicated SPF value, though the use of the indicated SPF value is not essential, and the suitability may be evaluated on the basis of the estimated SPF value regardless of the indicated SPF value.

<Additional Example 2>

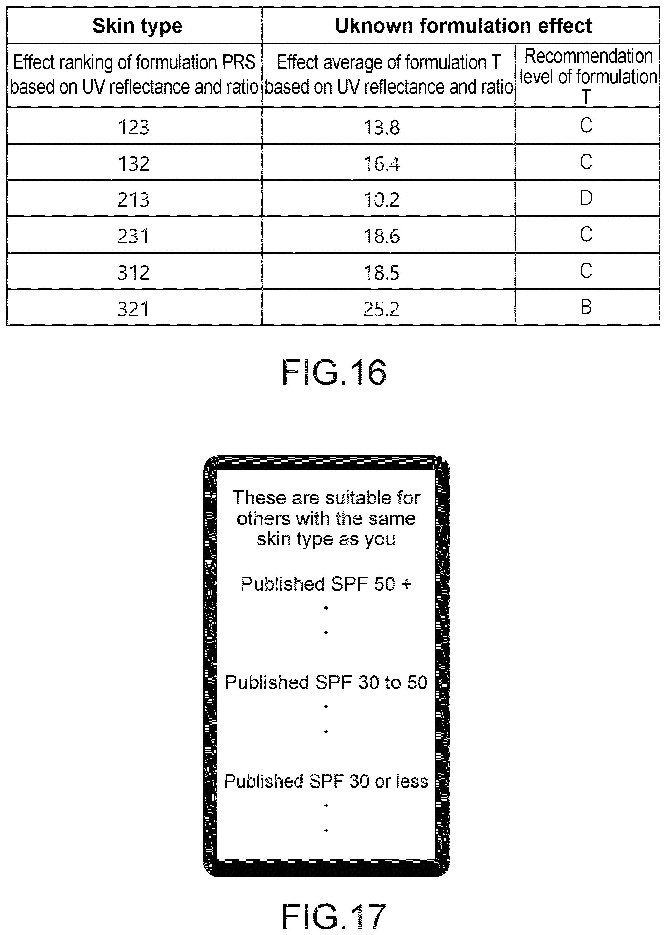

Fig. 16 is a view showing a result of predicting unknown effects of other topical skin preparations from measurement results of suitability of certain topical skin preparations in another embodiment of the present invention.

As shown in the figure, the subjects were classified into six clusters (skin types) on the basis of ranking of the UV protection effect estimated on the basis of the UV reflectances of Formulations P, R, and S and the average of the UV protection effects (SPF values) estimated on the basis of the UV reflectance of Formulation T was calculated for each cluster.

It should be noted that in the example of the figure, the number of subjects was set to 110, the indicated SPF values of the formulations were both set to 50+, application methods for the formulations were set to application with fingers, the application site was set to cheeks, the application area was set to 8 cm$^2$, and the application amount was set to 8 mg.

As it can be seen from the figure, an average of effects of Formulation T differs for each cluster (skin type). For example, the skin type "123" in the figure shows a subject cluster for which the average of the UV protection effects (SPF values) was the order of Formulations P, R, and S. Moreover, the skin type "321" shows a subject cluster for which the average of the UV protection effects (SPF values) was the order of Formulation S, R, and P.

In accordance with this skin type,
Formulations P, R, and S can be presented to each subject as a recommended product or non-recommended product on the basis of the effect order thereof. Moreover, accordingly, each subject can know the subject's skin type. In addition, in accordance with that skin type, suitability (SPF value) for the skin of unknown Formulation T different from Formulations P, R, and S used for classifying the above-mentioned skin types can be evaluated and predicted for a new user and product-recommending information or product-not-recommending information can be sent to the information processing terminal of the user for displaying that information.

Fig. 17 is a view showing an example of display on the information processing terminal of the product-recommending information based on the skin type. As shown in the figure, for example, information regarding a formulation recommend to a user having a specific skin type is displayed for each (range of) indicated SPF.

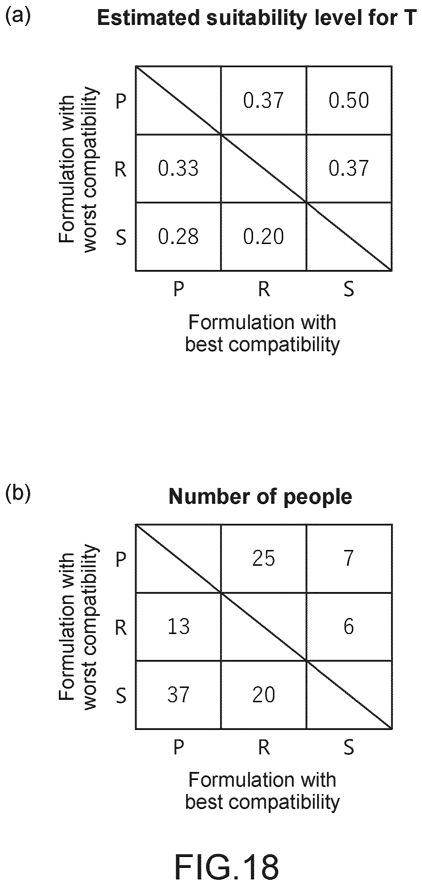

Moreover, as shown in Fig. 18, the skin type can also be represented as a map using the suitability level for the skin of the user with respect to such Formulations P, R, and S and displayed on the information processing terminal of the user. The six skin types are classified by ranking of the UV protection effect (estimated SPF value) of three Formulations P, R, and S for the skin of the user. Therefore, the skin type is determined by a combination of a formulation with the highest suitability level (UV protection effect) for the user and a formulation with the lowest suitability level among such Formulations P, R, and S. By forming a matrix with these formulations, the six skin types can be represented as a map with six cells.

The figure (a) displays an estimation suitability level with respect to Formulation T of the six skin types as the map. Each value in the map shows the ratio of the estimated SPF value to the indicated SPF value (50+) for each skin type. Accordingly, the user having each skin type can know a suitability level of Formulation T for the user on the basis of the values on the map.

The figure (b) displays the number of people classified into each of the six skin types among the subjects as the map. This allows users each having a skin type to see what proportion of the total population their skin type represents and what proportion other skin types account for on the basis of the values on the map.

It should be noted that since each region of such a map indicates the skin type, a hyperlink may be set on that map so that information related to a formulation recommend to the user having the skin type by selecting and operating any one of the regions is displayed.

<Additional Example 3>

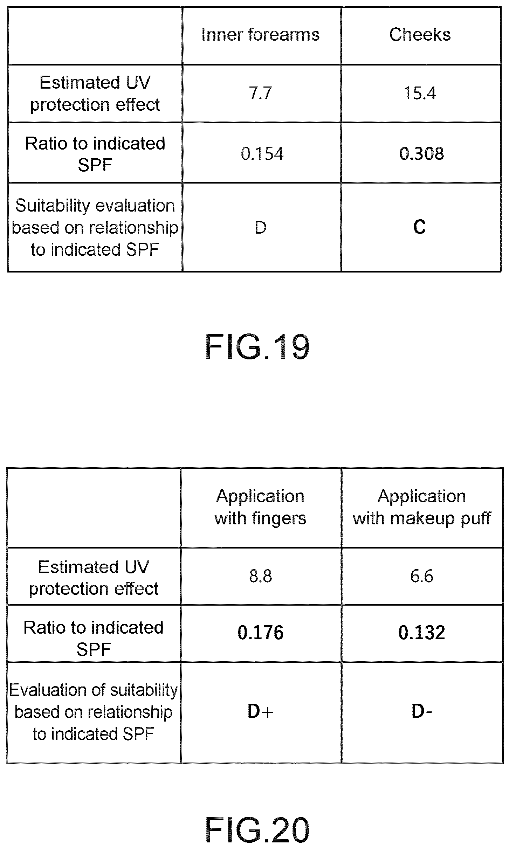

In the above-mentioned embodiments, it is also possible to compare and present the suitability level of each formulation for the skin of the user between different body sites. Fig. 19 is a view showing suitability evaluation based on a relationship between the estimated SPF value and the indicated SPF value between different body sites.

The example of the figure shows an evaluation result in a case where Formulation A with an amount of 16 mg in the form of gel with an indicated SPF of 50+ was uniformly applied to a region of 16 cm$^2$ of each of inner forearms and cheeks with fingers.

As it can be seen from the figure, the ratio of the estimation SPF to the indicated SPF in a case where it was applied to the cheeks is significantly higher than in a case where it was applied to the inner forearms. It can be seen that the effect differs depending on the application site even for the same formulation. It is possible to present information related to the formulation suitable for each site for the user who seeks the UV protection effect on the basis of such information.

<Additional Example 4>

In the above-mentioned embodiments, it is also possible to compare and present the suitability level of each formulation for the skin of the user between different application methods (tools). Fig. 20 is a view showing suitability evaluation based on the relationship between the estimated SPF value and the indicated SPF value between different application methods.

In the example of the figure, as a method of applying to the face of the user, application with fingers and application with a makeup puff are executed. Conditions related to other formulations and the application area are similar to those of Additional Example 3 described above.

As it can be seen from the figure, the ratio of the estimation SPF to the indicated SPF in a case where it was applied with fingers is higher than in a case where it was applied with a makeup puff. It can be seen that the effect differs depending on the application method (tool) even for the same formulation. It is possible to present information related to the application method (tool) suitable for the user who seeks the UV protection effect on the basis of such information.

It should be noted that a threshold for classifying the suitability level into evaluation values (scales), such as A to E, may differ between a case of comparing the suitability for the skin level (UV protection effect) between different formulations as in Additional Example 1 or 2 and a case of comparing the suitability level between different sites or tools with respect to the same formulation as in Additional Example 3 or 4.

Reference Signs List

| 10 | skin |
| 20 | topical skin preparation with sunscreen effect |
| 30 | UV camera |
| 50 | lighting unit |
| 70 | detection unit |
| 100 | information processing apparatus |
| 130 | obtaining unit |
| 150 | estimation unit |
| 160 | evaluation unit |
| 170 | input unit |
| 180 | display unit |
| 200 | evaluation apparatus |
| 300 | information processing apparatus |
| 330 | obtaining unit |
| 350 | estimation unit |
| 360 | evaluation unit |
| 370 | input unit |
| 380 | display unit |
| 400 | evaluation system |

**Claims**

1.  An evaluation method, comprising:

    irradiating a skin of a user to which a topical skin preparation with a sunscreen effect is applied with UV light and obtaining reflection light of the irradiated UV light; and
    evaluating suitability of the topical skin preparation for the skin of the user on a basis of the reflection light.

2.  The evaluation method according to claim 1, further comprising:

    estimating a UV protection value of the topical skin preparation for the skin of the user on a basis of the reflection light; and
    evaluating suitability of the topical skin preparation for the skin of the user by using the UV protection value.

3.  The evaluation method according to claim 1 or 2, wherein
    the suitability includes uniformity of a coating film of the topical skin preparation created on the skin of the user when the topical skin preparation is applied to the skin of the user.

4.  The evaluation method according to claim 1 or 2, wherein
    the suitability includes uniform applicability of the topical skin preparation to the skin of the user.

5.  The evaluation method according to claim 1 or 2, wherein
    the topical skin preparation comprises multiple kinds of topical skin preparations and each of the multiple kinds of topical skin preparations is applied to the skin of the user, the method further comprising:

    irradiating the skin of the user with UV light and obtaining reflection light of the irradiated UV light for each of the topical skin preparations applied to the skin of the user;
    estimating a UV protection value of the topical skin preparation for the skin of the user on a basis of the reflection light for each of the topical skin preparations; and

executing evaluation to extract the topical skin preparation with high suitability by using the estimated multiple kinds of UV protection values.

6. The evaluation method according to claim 1 or 2, wherein
the topical skin preparation comprises multiple kinds of topical skin preparations and each of the multiple kinds of topical skin preparations is applied to different regions of the skin of the user, the method further comprising:

irradiating the skin of the user with UV light and obtaining reflection light for each of the topical skin preparations applied to the skin of the user;
estimating a UV protection value of the topical skin preparation for the skin of the user on a basis of the reflection light for each of the topical skin preparations; and
executing evaluation to extract the topical skin preparation with high suitability by using the estimated multiple kinds of UV protection values.

7. The evaluation method according to claim 1 or 2, wherein
the topical skin preparation has a published UV protection value previously published, the method **characterized by** further comprising:

estimating a UV protection value of the topical skin preparation for the skin of the user on a basis of the reflection light; and
evaluating suitability of the topical skin preparation for the skin of the user by using the estimated UV protection value and the published UV protection value.

8. The evaluation method according to claim 1 or 2, wherein
the topical skin preparation has a published UV protection value previously published and the published UV protection value is SPF 40 or more.

9. The evaluation method according to claim 1 or 2, wherein
the topical skin preparation has a published UV protection value previously published and the published UV protection value is less than SPF 40.

10. The evaluation method according to claim 1 or 2, further comprising

irradiating the skin of the user to which multiple kinds of the topical skin preparations are applied with UV light, and obtaining reflection light, wherein
the multiple kinds of topical skin preparations have published UV protection values previously published, respectively, and
the published UV protection values of at least some of the multiple kinds of topical skin preparations are same.

11. The evaluation method according to claim 1 or 2, further comprising:

setting a plurality of application patterns depending on application amounts of the topical skin preparation on a bare skin of the user;
irradiating the skin of the user with UV light and obtaining reflection light for each of the application patterns; and
estimating a UV protection value of the topical skin preparation for the skin of the user on a basis of the reflection light for each of the application patterns.

12. The evaluation method according to any one of claims 1 to 11, further comprising:

obtaining an answer to a questionnaire from the user;
estimating a UV protection value of the topical skin preparation for the skin of the user on a basis of the reflection light; and
evaluating suitability of the topical skin preparation for the skin of the user by using the UV protection value and the answer.

13. The evaluation method according to claim 1 or 2, further comprising
outputting information indicating the evaluated suitability.

**14.** The evaluation method according to claim 13, further comprising:
transmitting, to an information processing terminal of the user,

information that recommends a topical skin preparation, which is evaluated to have high suitability for the skin of the user, to the user, or
information that does not recommend a topical skin preparation, which is evaluated to have low suitability for the skin of the user, to the user.

**15.** The evaluation method according to claim 13, further comprising:

classifying users into multiple skin types on a basis of the suitability of multiple kinds of topical skin preparations for skins of the users; and
transmitting, to an information processing terminal of the user, information that recommends a topical skin preparation to the user or information that does not recommend a topical skin preparation to the user depending on the skin type.

**16.** The evaluation method according to claim 15, further comprising:

evaluating suitability of a different topical skin preparation for the skin of the user for each skin type of users, the different topical skin preparation being different from the multiple kinds of topical skin preparations used to classify users into multiple skin types; and
transmitting, to an information processing terminal of the user, information that recommends the different topical skin preparation to a different user or information that does not recommend the different topical skin preparation to the user on a basis of the evaluation result.

**17.** The evaluation method according to claim 7, wherein

evaluating suitability of the topical skin preparation for the skin of the user higher as a value obtained by extracting the published UV protection value from the estimated UV protection value is larger, or as a ratio of the estimated UV protection value to the published UV protection value is larger; and
transmitting, to an information processing terminal of the user, on a basis of the value or ratio,

information that recommends a topical skin preparation, which is evaluated to have high suitability for the skin of the user, to the user, or
information that does not recommend a topical skin preparation, which is evaluated to have low suitability for the skin of the user, to the user.

**18.** An evaluation apparatus, comprising:

an obtaining means for irradiating a skin of a user to which a topical skin preparation with a sunscreen effect is applied with UV light and obtaining reflection light of the irradiated UV light; and
an evaluation means for evaluating suitability of the topical skin preparation with the sunscreen effect for the skin of the user by using the reflection light.

**19.** The evaluation apparatus according to claim 13, further comprising

an estimation means for estimating a UV protection value of the topical skin preparation for the skin of the user on a basis of the reflection light, wherein
suitability of the topical skin preparation for the skin of the user is evaluated by using the UV protection value.

**20.** The evaluation apparatus according to claim 14, **characterized by** further comprising

an input means capable of inputting an answer to a questionnaire, wherein
the evaluation means evaluates the suitability by using the UV protection value and the answer input by the input means.

**21.** A program that causes an evaluation apparatus irradiates a skin of the user to which a topical skin preparation with a sunscreen effect is applied with UV light and evaluates suitability of the topical skin preparation with the sunscreen effect for the skin of the user by using reflection light of the irradiated UV light to operate, the program causing the evaluation apparatus to execute:

an obtaining step of irradiating a skin of a user to which a topical skin preparation with a sunscreen effect is applied with UV light and obtaining reflection light of the irradiated UV light; and
a step of evaluating suitability of the topical skin preparation for the skin of the user by using the reflection light.

22. A storage medium storing a program that causes an evaluation apparatus irradiates a skin of the user to which a topical skin preparation with a sunscreen effect is applied with UV light, and evaluates suitability of the topical skin preparation with the sunscreen effect for the skin of the user by using reflection light of the irradiated UV light to operate, the program causing the evaluation apparatus to execute:

a step of irradiating a skin of a user to which a topical skin preparation with a sunscreen effect is applied with UV light and obtaining reflection light of the irradiated UV light; and
a step of evaluating suitability of the topical skin preparation for the skin of the user by using the reflection light.

23. An evaluation system, comprising:

a UV light irradiate means for irradiating a skin of a user to which a topical skin preparation with a sunscreen effect is applied with UV light;
a detection means for detecting reflection light of the irradiated UV light;
an obtaining means for obtaining the detected reflection light of the UV light; and
an evaluation means for evaluating suitability of the topical skin preparation with the sunscreen effect for the skin of the user by using the reflection light.

FIG.1

FIG.2

EP 4 744 579 A1

Lighting unit 50

Detection unit 70
(including UV camera)

Information processing apparatus 100

Input unit 170

Obtaining unit 130

Estimation unit 150

Evaluation unit 160

Display unit 180

Evaluation apparatus 200

$$I(\lambda)(1 - S(\lambda))(1 - \beta(\lambda))T_S(\lambda)R_S(\lambda)T_S(\lambda)$$

$$I(\lambda)(1 - S(\lambda))T_S(\lambda)\beta(\lambda)T_S(\lambda)$$

$$I(\lambda)$$

$$I(\lambda)S(\lambda)$$

20 — $T_S(\lambda)$

10 — $R_S(\lambda)$

# FIG.3

FIG.4

EP 4 744 579 A1

50

70

150

Lighting
unit

Detection unit
(including UV
camera 30)

Estimation
unit

Reflection
light

Incident
light

20

10

FIG.5

| Formulation | | A | D | E | F |
|---|---|---|---|---|---|
| Form | | Gel | Gel | Gel | Emulsion |
| Formulation type | | O/W | O/W | O/W | W/O |
| Indicated SPF | | 30 | 50 | 50+ | 50+ |
| Application amount(mg/cm$^2$) | | 1 | 1 | 1 | 1 |
| Application part | | Face | Face | Face | Face |
| Estimated SPF value | Subject 1 | 10.5 | 12.1 | 20.0 | 15.4 |
| | Subject 2 | 24.4 | - | 33.9 | 20.2 |
| | Subject 3 | 21.1 | - | - | 52.5 |
| | Subject 4 | 22.7 | 11.4 | - | - |
| | Subject 5 | - | 11.1 | 34.9 | - |
| | Subject 6 | - | 10.1 | 13.9 | 34.3 |
| | Subject 7 | 20.5 | 16.7 | 22.1 | 28.5 |

# FIG.6

(a)

Subject 1

(b)

Subject 7

# FIG.7

| Formulation | | A | D | E | F |
|---|---|---|---|---|---|
| Form | | Gel | Gel | Gel | Emulsion |
| Formulation type | | O/W | O/W | O/W | W/O |
| Indicated SPF | | 30 | 50 | 50+ | 50+ |
| Application amount($mg/cm^2$) | | 1 | 1 | 1 | 1 |
| Application part | | Face | Face | Face | Face |
| UV protection value | Subject 1 | 0.35 | 0.24 | 0.33 | 0.26 |
| | Subject 2 | 0.81 | - | 0.56 | 0.34 |
| | Subject 3 | 0.70 | - | - | 0.88 |
| | Subject 4 | 0.76 | 0.23 | - | - |
| | Subject 5 | - | 0.22 | 0.58 | - |
| | Subject 6 | - | 0.20 | 0.23 | 0.57 |
| | Subject 7 | 0.68 | 0.33 | 0.37 | 0.47 |

# FIG.8

| Formulation | | P | Q | R | S |
|---|---|---|---|---|---|
| Form | | Gel | Gel | Gel | Emulsion |
| Formulation type | | O/W | O/W | O/W | W/O |
| Indicated SPF | | 50+ | 50+ | 50+ | 50+ |
| Application amount(mg/cm$^2$) | | 1 | 1 | 1 | 1 |
| Application part | | Face | Face | Face | Face |
| Estimated SPF value | Subject 8 | 26.1 | 38.6 | 18.5 | 12.4 |
| | Subject 9 | 37.8 | 49.2 | 23.6 | 20.1 |
| | Subject 10 | 19.0 | 65.3 | 42.6 | 9.6 |
| | Subject 11 | 21.9 | 23.5 | 79.7 | 47.3 |
| | Subject 12 | 42.5 | 19.4 | 10.6 | 33.1 |

# FIG.9

| Formulation | | A | B | C | D | E | F |
|---|---|---|---|---|---|---|---|
| Form | | Gel | Gel | Emulsion | LFD | Makeup base | LFD |
| Formulation type | | O/W | O/W | W/O | W/O | W/O | W/O |
| Indicated SPF | | 30 | 15 | 27 | 28 | 28 | 32 |
| Application amount(mg/cm$^2$) | | 2 | 2 | 2 | 2 | 2 | 2 |
| Application part | | Back | Back | Back | Back | Back | Back |
| Estimated SPF value | Subject 13 | 23.1 | 11.1 | 22.3 | | | |
| | Subject 14 | 20.1 | 19.8 | 20.2 | | | |
| | Subject 15 | 29.8 | 17.4 | 21.4 | | | |
| | Subject 16 | 30.6 | 12.7 | 17.8 | | | |
| | Subject 17 | | | | 36.4 | 22.2 | 22.1 |
| | Subject 18 | | | | 23.1 | 23.3 | 27.6 |
| | Subject 19 | | | | 28.1 | 18.9 | 25.7 |

LFD: Liquid foundation

# FIG.10

Subject 21

FIG.11

| Formulation | F | A | B | C |
|---|---|---|---|---|
| Form | Emulsion | Gel | Emulsion | Emulsion |
| Formulation type | W/O | O/W | W/O | W/O |
| Indicated SPF | 30 | 50+ | 50+ | 50+ |
| Application amount (mg/cm$^2$) | 1 | 1 | 1 | 1 |
| Application part | Face | Face | Face | Face |
| Estimated SPF | 22.16 | 13.19 | 13.52 | 27.80 |
| Coefficient of variation (uniformity evaluation indication) | 0.218 | 0.113 | 0.105 | 0.143 |

# FIG.12

EP 4 744 579 A1

# FIG.13

| Formulation | | A |
|---|---|---|
| Form | | Gel |
| Formulation type | | O/W |
| Indicated SPF | | 30 |
| Application amount(mg/cm$^2$) | | 1 |
| Application part | | Face |
| Estimated SPF | Subject 1 | 10.5 |
| Estimated SPF/published SPF | | 0.35 |

## FIG.14

Product for you
○○
Published SPF xx

Product not for you
△△
Published SPF xx

## FIG.15

| Skin type | Uknown formulation effect | |
|---|---|---|
| Effect ranking of formulation PRS based on UV reflectance and ratio | Effect average of formulation T based on UV reflectance and ratio | Recommendation level of formulation T |
| 123 | 13.8 | C |
| 132 | 16.4 | C |
| 213 | 10.2 | D |
| 231 | 18.6 | C |
| 312 | 18.5 | C |
| 321 | 25.2 | B |

## FIG.16

These are suitable for
others with the same
skin type as you

Published SPF 50 +

·

·

Published SPF 30 to 50

·

·

Published SPF 30 or less

·

·

## FIG.17

(a) **Estimated suitability level for T**

(b) **Number of people**

# FIG.18

| | Inner forearms | Cheeks |
|---|---|---|
| Estimated UV protection effect | 7.7 | 15.4 |
| Ratio to indicated SPF | 0.154 | 0.308 |
| Suitability evaluation based on relationship to indicated SPF | D | C |

## FIG.19

| | Application with fingers | Application with makeup puff |
|---|---|---|
| Estimated UV protection effect | 8.8 | 6.6 |
| Ratio to indicated SPF | 0.176 | 0.132 |
| Evaluation of suitability based on relationship to indicated SPF | D+ | D- |

## FIG.20

## INTERNATIONAL SEARCH REPORT

| International application No. |
| --- |
| **PCT/JP2024/025389** |

| A. | CLASSIFICATION OF SUBJECT MATTER |
| --- | --- |

*A61B 5/00*(2006.01)i; *A61K 8/00*(2006.01)i; *A61Q 17/04*(2006.01)i; *G01N 21/33*(2006.01)i; *G01N 21/47*(2006.01)i
FI:  A61B5/00 M; A61B5/00 101A; A61K8/00; A61Q17/04; G01N21/33; G01N21/47 B

According to International Patent Classification (IPC) or to both national classification and IPC

| B. | FIELDS SEARCHED |
| --- | --- |

Minimum documentation searched (classification system followed by classification symbols)

A61B5/00; A61K8/00; A61Q17/04; G01N21/33; G01N21/47

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2010/113961 A1 (SHISEIDO COMPANY, LTD.) 07 October 2010 (2010-10-07) paragraphs [0036]-[0037], [0063]-[0080], [0086]-[0087], [0107]-[0134], [0151], fig. 7A-8 | 1-6, 8-16, 18-23 |
| A | | 7, 17 |
| X | JP 2017-146211 A (KAO CORPORATION) 24 August 2017 (2017-08-24) abstract, paragraphs [0024]-[0041] | 1-2, 5-6, 8-16, 18-23 |
| A | US 2021/0102839 A1 (BEIJING YOUCAI TECH CO., LTD.) 08 April 2021 (2021-04-08) abstract, paragraphs [0015]-[0049], fig. 1-3 | 1-23 |
| A | KOBYLINSKI, S., et al., Noninvasive measurement of the 308 nm LED-based UVB protection factor of sunscreens, Journal of biophotonics, April 2021, vol. 14, no. 4, e202000453, pp. 1-11, <DOI: 10.1002/jbio.202000453> abstract, fig. 4-7, table. 3 | 1-23 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **09 September 2024** | **17 September 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

## INTERNATIONAL SEARCH REPORT

| | International application No. |
|---|---|
| | **PCT/JP2024/025389** |

### C.    DOCUMENTS CONSIDERED TO BE RELEVANT

| Category* | Citation of document, with indication, where appropriate, of the relevant passages | Relevant to claim No. |
|---|---|---|
| A | 日本化粧品工業連合会SPF測定法基準の改定について, 05 November 2011, <URL: https://jcia.org/user/common/download/business/guideline/spf/SPF-jcia-h231005.pdf>, non-official translation (Revision of the Japan Cosmetic Industry Association's SPF measurement standard)<br>    entire text | 1-23 |
| A | 日本化粧品工業連合会SPF測定法基準について, 21 February 2020, <URL: https://jcia.org/user/common/download/business/guideline/spf/SPF-jcia-r020221.pdf>, non-official translation (Japan Cosmetic Industry Association's SPF measurement standard)<br>    entire text | 1-23 |

Form PCT/ISA/210 (second sheet) (July 2022)

## INTERNATIONAL SEARCH REPORT
### Information on patent family members

| International application No. |
| --- |
| **PCT/JP2024/025389** |

| Patent document cited in search report | | | Publication date (day/month/year) | Patent family member(s) | Publication date (day/month/year) |
| --- | --- | --- | --- | --- | --- |
| WO | 2010/113961 | A1 | 07 October 2010 | US 2012/0022472 A1<br>paragraphs [0050]-[0051],<br>[0077]-[0094], [0100]-[0101],<br>[0119]-[0150], [0168], fig.<br>7A-8<br>EP 2416144 A1<br>CN 102369429 A<br>KR 10-2012-0002571 A | |
| JP | 2017-146211 | A | 24 August 2017 | (Family: none) | |
| US | 2021/0102839 | A1 | 08 April 2021 | WO 2019/227601 A1<br>CN 108760685 A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017146211 A